(19) Europäisches Patentamt
European Patent Office
Office européen des brevets

(11) EP 4 148 070 A1

(12) **EUROPEAN PATENT APPLICATION**
published in accordance with Art. 153(4) EPC

(43) Date of publication:
**15.03.2023 Bulletin 2023/11**

(21) Application number: **20934690.7**

(22) Date of filing: **03.05.2020**

(51) International Patent Classification (IPC):
***C07K 19/00*** (2006.01) ***C07K 1/107*** (2006.01)
***A61K 38/07*** (2006.01) ***A61P 35/00*** (2006.01)

(52) Cooperative Patent Classification (CPC):
**A61K 38/07; A61P 35/00; C07K 1/107; C07K 19/00**

(86) International application number:
**PCT/CN2020/088564**

(87) International publication number:
**WO 2021/223048 (11.11.2021 Gazette 2021/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Shanghai Miracogen Inc.**
**Shanghai 201203 (CN)**

(72) Inventors:
- **HU, Chaohong**
**Shanghai 201203 (CN)**
- **LI, Hu**
**Shanghai 201203 (CN)**
- **XIAO, Lili**
**Shanghai 201203 (CN)**
- **LIU, Wenchao**
**Shanghai 201203 (CN)**

(74) Representative: **Lavoix**
**Bayerstraße 83**
**80335 München (DE)**

(54) **ANTIBODY-DRUG CONJUGATE AND PREPARATION THEREOF**

(57) Provided are an anti-CD20 antibody-drug conjugate, a preparation comprising the antibody-drug conjugate, a composition comprising the antibody-drug conjugate, and a pharmaceutical use of the antibody-drug conjugate.

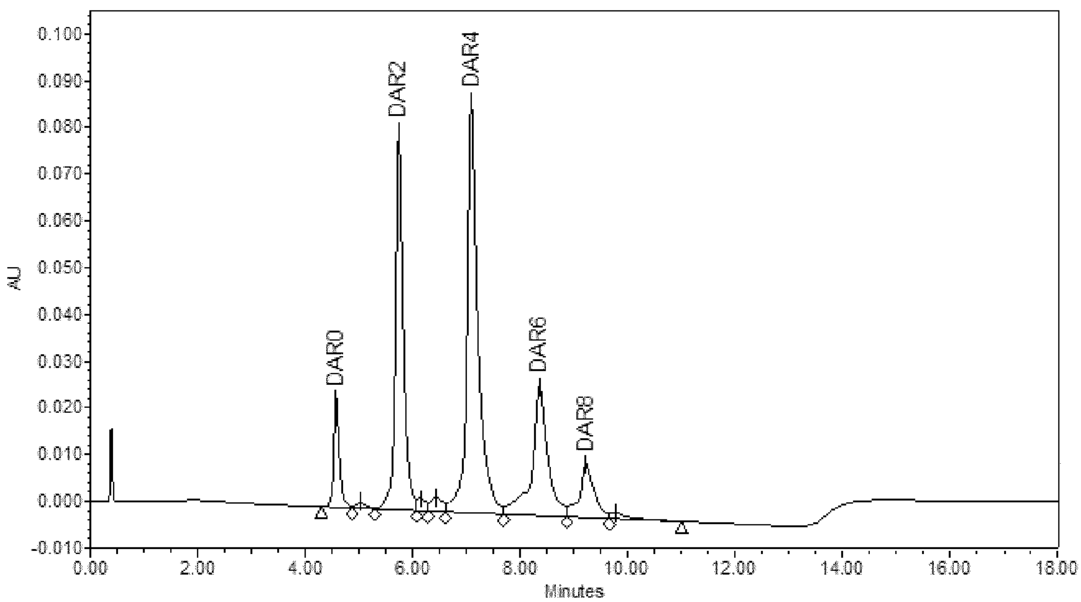


**Figure 1**

EP 4 148 070 A1

## Description

### Technical Field

The present invention relates to the field of biomedicine, in particular to an antibody-drug conjugate, a preparation comprising the antibody-drug conjugate, a composition comprising the antibody-drug conjugate, and medicinal use of the antibody-drug conjugate.

### Background Art

Currently, the treatment of non-Hodgkin's lymphoma (NHL) includes traditional surgery, chemotherapy, radiotherapy, bone marrow or hematopoietic stem cell transplantation, immune and targeted therapy. Traditional chemotherapy has a certain curative effect on NHL patients, but it has serious systemic side-effects, poor tolerance, and easy recurrence, which limit its application in patients. Targeted drugs, especially antibody drugs, have attracted much attention due to their remarkable curative effects and minimal systemic side-effects during therapeutic intervention.
In recent years, a number of CD20-targeted monoclonal antibodies have been developed (e.g., rituximab, Rituxan®), and their anti-tumor activity has been verified in clinical trials.
However, with the deepening of clinical application, the limitations of rituximab treatment have gradually emerged, that is, there are patients with primary and acquired resistance to the drug during the treatment process, resulting in reduction or invalidation in the therapeutic effect of rituximab. In addition, the existing CD20-targeted monoclonal antibodies are not ideal in the terms of effective rate of treating tumors, and the combination with chemotherapeutic drugs is required to improve the anti-tumor effects, which undoubtedly increases the risk of toxicity. From this point of view, the marketed CD20-targeting monoclonal antibodies represented by rituximab are far from meeting the needs of clinical practice for the treatment of relapsed or refractory B-cell NHL (including DLBCL and FL, etc.).
In view of the aforementioned problems of the existing CD20-targeting monoclonal antibodies, the main purpose of developing CD20-targeting ADC drug ADC-1 is to provide a treatment method with better efficacy, lower toxic and side effects, and less drug resistance.

### Contents of the present invention

The inventors of the present invention have prepared an anti-CD20 antibody-drug conjugate through a lot of experiments and creative work, and confirmed that it has good biological activity and formulation stability, thereby completing the present invention.
To this end, in the first aspect of the present invention, the present invention provides an antibody-drug conjugate, the antibody-drug conjugate having the structure shown in Formula I,

$$\text{Ab-(L-D)}_p \qquad \text{Formula I}$$

wherein:

Ab represents an anti-CD20 monoclonal antibody, and the anti-CD20 monoclonal antibody is any antibody targeting CD20, such as rituximab or a biosimilar thereof;

D represents a cytotoxic agent, and the cytotoxic agent is Monomethyl auristatin E (MMAE);

L represents a linker for linking the anti-CD20 monoclonal antibody with the cytotoxic agent, and the linker is 6-maleimidocaproyl-valine-citrulline-p-aminobenzyloxycarbonyl (MC-vc-PAB);

p is 3.6 to 4.0.

In some embodiments, p is 3.7 to 3.9.
In some embodiments, p is 3.8.
In some embodiments, the L-D in Formula I is vc-MMAE, and has a structure shown in the following formula:

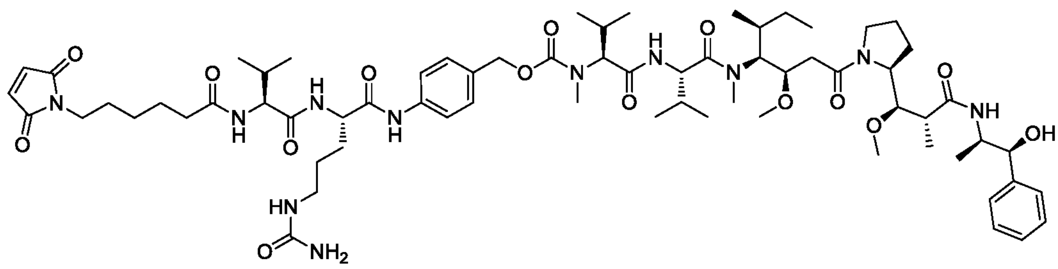

.

In some embodiments, the structure of the antibody-drug conjugate has a structure shown in the following formula:

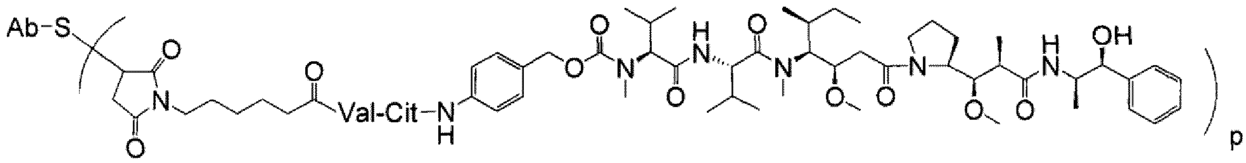

wherein:

Ab represents an anti-CD20 monoclonal antibody, the anti-CD20 monoclonal antibody is any antibody targeting CD20, such as rituximab or a biosimilar thereof,

p is 3.6 to 4.0.

In some embodiments, p is 3.7 to 3.9.
In some embodiments, p is 3.8.
In the second aspect of the present invention, the present invention provides an antibody-drug conjugate preparation, which comprises:

an antibody-drug conjugate, which has a concentration of 1 to 60 mg/mL (e.g., 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 13 mg/mL, 15 mg/mL, 17 mg/mL, 19 mg/mL, 20 mg/mL, 25 mg/mL, 30 mg/mL, 35 mg/mL, 40 mg/mL, 45 mg/mL, 50 mg/mL, 55 mg/mL, or 60 mg/mL);

a histidine buffer, having a concentration of 5 to 35 mM (e.g., 5 mM, 8 mM, 9 mM, 10 mM, 11 mM, 12 mM, 15 mM, 20 mM, 25 mM, 30 mM or 35 mM), and a pH of 5.0 to 6.2 (e.g., 5.0, 5.1, 5.2, 5.3, 5.4, 5.5, 5.6, 5.7, 5.8, 5.9, 6.0, 6.1 or 6.2);

sucrose, having a concentration of 2% to 10% (e.g., 2%, 3%, 4%, 5%, 6%, 7%, 8%, 9% or 10%); and

Tween-80, having a concentration of 0.01% to 0.1% (e.g., 0.01%, 0.015%, 0.02%, 0.025%, 0.03%, 0.031%, 0.032%, 0.033%, 0.034%, 0.035%, 0.036%, 0.037%, 0.038%, 0.039%, 0.04%, 0.045%, 0.05%, 0.055%, 0.06%, 0.065%, 0.07%, 0.08%, 0.09% or 0.1%);

the antibody-drug conjugate has a structure shown in Formula II,

$Ab\text{-}(L\text{-}D)_q$ Formula II

wherein:

Ab represents an anti-CD20 monoclonal antibody, and the anti-CD20 monoclonal antibody is any antibody targeting CD20, such as rituximab or a biosimilar thereof;
D represents a cytotoxic agent, and the cytotoxic agent is Monomethyl auristatin E (MMAE);
L represents a linker for linking the anti-CD20 monoclonal antibody with the cytotoxic agent, and the linker is 6-maleimidocaproyl-valine-citrulline-p-aminobenzyloxycarbonyl (MC-vc-PAB);
q is 3.3 to 4.3 (e.g., 3.3, 3.4, 3.5, 3.6, 3.7, 3.8, 3.9, 4.0, 4.1, 4.2 or 4.3).

In some embodiments, q is 3.6 to 4.0.
In some embodiments, q is 3.7 to 3.9.
In some embodiments, q is 3.8.
In some embodiments, the antibody-drug conjugate has a concentration of 1 to 20 mg/mL.
In some embodiments, the antibody-drug conjugate has a concentration of 1 to 10 mg/mL.
In some embodiments, the antibody-drug conjugate has a concentration of 3 to 7 mg/mL.
In some embodiments, the antibody-drug conjugate has a concentration of 5 mg/mL.
In some embodiments, the histidine buffer has a concentration of 5 to 15 mM.
In some embodiments, the histidine buffer has a concentration of 8 to 12 mM.
In some embodiments, the histidine buffer has a concentration of 10 mM.
In some embodiments, the histidine buffer has a pH of 5.8 to 6.2.
In some embodiments, the histidine buffer has a pH of 5.6 to 6.0.
In some embodiments, the histidine buffer has a pH of 5.4 to 6.0.
In some embodiments, the histidine buffer has a pH of 5.4 to 6.2.
In some embodiments, the sucrose has a concentration of 3% to 9%.
In some embodiments, the sucrose has a concentration of 4% to 8%.
In some embodiments, the sucrose has a concentration of 5% to 7%.
In some embodiments, the sucrose has a concentration of 6%.
In some embodiments, the Tween-80 has a concentration of 0.02% to 0.06%.
In some embodiments, the Tween-80 has a concentration of 0.02% to 0.05%.
In some embodiments, the Tween-80 has a concentration of 0.03% to 0.04%.
In some embodiments, the Tween-80 has a concentration of 0.035%.
In some embodiments, the preparation comprises:

the antibody-drug conjugate, having a concentration of 5 mg/mL;

the histidine buffer, having a concentration of 10 mM and a pH of 5.8;

6% sucrose; and

0.035% Tween-80.

It should be noted that "2% to 10% sucrose" represents mass volume concentration (w/v%), which means that in every 1000 mL of the antibody-drug conjugate preparation, the mass of sucrose is 20 to 100 g; "0.01% to 0.1% Tween-80" represents mass volume concentration (w/v%), and its meaning can be similarly understood with reference to the aforementioned "2% to 10% sucrose".
In addition, the concentration of "histidine buffer" refers to concentration of histidine and histidine hydrochloride, and its pH is obtained by adjusting the ratio of histidine to histidine hydrochloride.
In some embodiments, when the pH is 5.0 to 6.2 (e.g., 5.6 to 6.0), the antibody-drug conjugate preparation is stable for 6 weeks under conditions of 25±2°C/60%±5% RH.
In some embodiments, when the pH is 5.0 to 6.2 (e.g., 5.6 to 6.0), the antibody-drug conjugate preparation is colorless and clear in appearance when it is allowed to stand at 25°C for 6 weeks.
In some embodiments, when the pH is 5.0 to 6.2 (e.g., 5.6 to 6.0), the antibody-drug conjugate preparation does not change in pH when it is allowed to stand at 25°C for 6 weeks.
In some embodiments, when the pH is 5.0 to 6.2 (e.g., 5.6 to 6.0), the antibody-drug conjugate preparation does not change in concentration when it is allowed to stand at 25°C for 6 weeks.
In some embodiments, when the pH is 5.0 to 6.2 (e.g., 5.6 to 6.0), the antibody-drug conjugate preparation has a decrease of SEC monomer % content that is not greater than 2% (or not greater than 1%) when it is allowed to stand at 25°C for 6 weeks.
In some embodiments, when the pH is 5.0 to 6.2 (e.g., 5.6 to 6.0), the antibody-drug conjugate preparation has an increase of SEC polymer % content that is not greater than 2% (or not greater than 1%) when it is allowed to stand at 25°C for 6 weeks.
In some embodiments, when the pH is 5.0 to 6.2 (e.g., 5.6 to 6.0), the antibody-drug conjugate preparation has no significant change in DAR value when it is allowed to stand at 25°C for 6 weeks.
In some embodiments, when the pH is 5.0 to 6.2 (e.g., 5.6 to 6.0), the antibody-drug conjugate preparation is colorless and clear in appearance when it is allowed to stand at 40°C for 4 weeks.
In some embodiments, when the pH is 5.0 to 6.2 (e.g., 5.6 to 6.0), the antibody-drug conjugate preparation does not change in pH when it is allowed to stand at 40°C for 4 weeks.

In some embodiments, when the pH is 5.0 to 6.2 (e.g., 5.6 to 6.0), the antibody-drug conjugate preparation does not change in concentration when it is allowed to stand at 40°C for 4 weeks.

In some embodiments, when the pH is 5.0 to 6.2 (e.g., 5.6 to 6.0), the antibody-drug conjugate preparation has a decrease of SEC monomer % content that is not greater than 5% (or not greater than 4%) when it is allowed to stand at 40°C for 4 weeks.

In some embodiments, when the pH is 5.0 to 6.2 (e.g., 5.6 to 6.0), the antibody-drug conjugate preparation has an increase of SEC polymer % content that is not greater than 5% (or not greater than 4%) when it is allowed to stand at 40°C for 4 weeks.

In some embodiments, when the pH is 5.0 to 6.2 (e.g., 5.6 to 6.0), the antibody-drug conjugate preparation has no significant change in DAR value when it is allowed to stand 40°C for 4 weeks.

It should be noted that the term "no significant change" or "no change" refers to no statistically significant change.

In addition, it should be noted that the above-mentioned "antibody-drug conjugate" refers to a composition containing ADC molecules with the same or different DAR values.

Specifically, the present invention provides a composition comprising a plurality of anti-CD20 ADC molecules. In certain instances, each ADC in the composition described herein comprises the same number of one or more drug molecules. In other instances, each ADC in the composition described herein comprises a different number of one or more drug molecules.

In the antibody-drug conjugate described herein, each anti-CD20 antibody can be conjugated with 1, 2, 3, 4, 5, 6, 7, 8 or more (preferably 2, 4, 6 or 8, more preferably 2 or 4) drug molecules.

The above drug-to-antibody ratio (DAR) refers to the molecular number of anticancer drug conjugated to anti-CD20 antibody. The molecular number of anticancer drug contained in the ADC described herein is usually an integer, when the molecular number of anticancer drug contained in the ADC described herein (e.g., p in Formula I, or q in Formula II) is a fraction, the fraction refers to the average molecular number of anticancer drug conjugated to each anti-CD20 antibody in a composition comprising a plurality of ADC molecules.

In the third aspect of the present invention, the present invention provides a method for preparing the aforementioned antibody-drug conjugate preparation, comprising:

performing a reduction reaction of an anti-CD20 monoclonal antibody with a reducing agent to obtain a reduced anti-CD20 monoclonal antibody, wherein the anti-CD20 monoclonal antibody is any antibody targeting CD20, such as rituximab or a biosimilar thereof;

performing a coupling reaction between the reduced anti-CD20 monoclonal antibody and vc-MMAE;

quenching the coupling reaction;

allowing the quenched coupling reaction product to undergo a buffer replacement to obtain the antibody-drug conjugate preparation, the antibody-drug conjugate preparation comprising:

the antibody-drug conjugate having a concentration of 1 to 60 mg/mL (e.g., 1 mg/mL, 2 mg/mL, 3 mg/mL, 4 mg/mL, 5 mg/mL, 6 mg/mL, 7 mg /mL, 8 mg/mL, 9 mg/mL, 10 mg/mL, 13 mg/mL, 15 mg/mL, 17 mg/mL, 19 mg/mL, 20 mg/mL, 25 mg/mL, 30 mg /mL, 35 mg/mL, 40 mg/mL, 45 mg/mL, 50 mg/mL, 55 mg/mL, or 60 mg/mL);

the histidine buffer having a concentration of 5 to 35 mM (e.g., 5 mM, 8 mM, 9 mM, 10 mM, 11 mM, 12 mM, 15 mM, 20 mM, 25 mM, 30 mM, or 35 mM), and a pH of 5.0 to 6.2 (e.g., 5.0, 5.1, 5.2, 5.3, 5.4, 5.5, 5.6, 5.7, 5.8, 5.9, 6.0, 6.1 or 6.2);

the sucrose having a concentration of 2% to 10% (e.g., 2%, 3%, 4%, 5%, 6%, 7%, 8%, 9% or 10%); and

the Tween-80 having a concentration of 0.01% to 0.1% (e.g., 0.01%, 0.015%, 0.02%, 0.025%, 0.03%, 0.031%, 0.032%, 0.033%, 0.034%, 0.035%, 0.036%, 0.037%, 0.038%, 0.039%, 0.04%, 0.045%, 0.05%, 0.055%, 0.06%, 0.065%, 0.07%, 0.08%, 0.09%, or 0.1%).

In some embodiments, the antibody-drug conjugate has a concentration of 1 to 20 mg/mL.

In some embodiments, the antibody-drug conjugate has a concentration of 1 to 10 mg/mL.

In some embodiments, the antibody-drug conjugate has a concentration of 3 to 7 mg/mL.

In some embodiments, the antibody-drug conjugate has a concentration of 5 mg/mL.

In some embodiments, the histidine buffer has a concentration of 5 to 15 mM.

In some embodiments, the histidine buffer has a concentration of 8 to 12 mM.

In some embodiments, the histidine buffer has a concentration of 10 mM.
In some embodiments, the histidine buffer has a pH of 5.8 to 6.2.
In some embodiments, the histidine buffer has a pH of 5.6 to 6.0.
In some embodiments, the histidine buffer has a pH of 5.4 to 6.0.
In some embodiments, the histidine buffer has a pH of 5.4 to 6.2.
In some embodiments, the sucrose has a concentration of 3% to 9%.
In some embodiments, the sucrose has a concentration of 4% to 8%.
In some embodiments, the sucrose has a concentration of 5% to 7%.
In some embodiments, the sucrose has a concentration of 6%.
In some embodiments, the Tween-80 has a concentration of 0.02% to 0.06%.
In some embodiments, the Tween-80 has a concentration of 0.02% to 0.05%.
In some embodiments, the Tween-80 has a concentration of 0.03% to 0.04%.
In some embodiments, the Tween-80 has a concentration of 0.035%.
In some embodiments, the reducing agent is DTT.
In some embodiments, the preparation method comprises:

1) 10 mg of anti-CD20 monoclonal antibody is taken, and subjected to replacement into a reducing buffer (25mM sodium borate, pH 8.0, 25mM NaCl, 5 mM EDTA) by using a 15 mL 30KD ultrafiltration device, and a total of three replacements are performed; a final volume of about 1 mL is obtained, the obtained solution is transferred to a new Eppendorf centrifuge tube (weighed), and weighed; the protein concentration is detected and the total protein is calculated;

2) DTT at 2.0 to 3.0 times moles is added to the antibody, incubated at room temperature for 2 hours, and mixed continuously; the obtained solution is subjected to replacement into a coupling buffer (50mM Tris, pH 7.2, 150 mM NaCl, 5 mM EDTA) by using a 15 mL 30KD ultrafiltration device, and a total of three replacements are performed; the concentrated solution is taken, its protein concentration is detected by $A_{280}$ (absorbance at 280 nm wavelength), and it is weighed to calculate the total protein; 10 μL sample is taken, and the number of free sulfhydryl groups is determined by using Ellman's method;

and the molar concentration of its free sulfhydryl groups is calculated by the following formula (wherein $A_{412}$ represents the absorbance at 412nm wavelength):

$$C_{thiol} = \frac{A412 \times 11.2}{b \times 14150} \ (M)$$

b: optical path length of cuvette (usually 1cm)

the number of moles of free sulfhydryl groups is calculated according to the molar concentration of free sulfhydryl groups and the volume of the total protein solution;

3) vc-MMAE (dissolved in DMSO) is added at 1.0 to 1.5 times the number of moles of free sulfhydryl groups to the reduced antibody, mixed well, and then reacted at room temperature for 2 hours with intermittent agitation; to the reaction system, N-acetylcysteine is added at 20 times the number of moles of vc-MMAE moles to the reaction solution, mixed well, and allowed to stand for 5 minutes;

4) the obtained solution in step 3) is subjected to replacement into a conjugate stock solution (10 mM histidine, 6% sucrose, 0.035% PS80, pH 5.8) by using a 15 mL 30KD ultrafiltration device, a total of three replacements are performed, and thus the antibody-drug conjugate preparation is obtained, and stored at 4°C.

In the fourth aspect of the present invention, the present invention provides a composition, which comprises the aforementioned antibody-drug conjugate, or the aforementioned antibody-drug conjugate preparation, or the antibody-drug conjugate preparation prepared by the aforementioned method.
In some embodiments, the composition further comprises at least one pharmaceutically acceptable carrier, diluent or excipient.
In some embodiments, the composition further comprises a known chemotherapeutic drug for the treatment of a tumor, and the chemotherapeutic drug can be, for example, doxorubicin (Adriamycin), cyclophosphamide and taxanes [paclitaxel (Taxol) and docetaxel (Taxotere)], capecitabine (Xeloda), gemcitabine (Gemzar), vinorelbine (Navelbine), tamoxifen, aromatase inhibitor (Arimidex, Femara, Aromasin), 5-FU/leucovorin, irinotecan (camptosar), oxaliplatin, cisplatin, carboplatin, estramustine, mitoxantrone (Novantrone), prednisone, vincristine (Oncovin), Doxorubicin, prednisone, etc., or a combination thereof.

In some embodiments, the composition further comprises an immunosuppressive agent selected from: (1) glucocorticoid, such as cortisone and prednisone; (2) microbial metabolite, such as cyclosporine and tacrolimus, etc.; (3) anti-metabolite, such as azathioprine and 6-mercaptopurine, etc.; (4) polyclonal and monoclonal anti-lymphocyte antibodies, such as anti-lymphocyte globulin and OKT3, etc.; (5) alkylating agent, such as cyclophosphamide. Specifically, the immunosuppressive agent is, for example, methylprednisolone, prednisone, azathioprine, Prograf, Zenapax, Simulect, cyclosporine, tacrolimus, rapamycin, mycophenolate, mizoribine, cyclophosphamide, Fingolimod, etc.

In the fifth aspect of the present invention, the present invention provides use of the aforementioned antibody-drug conjugate, or the aforementioned antibody-drug conjugate preparation, or the antibody-drug conjugate preparation prepared by the aforementioned method, or the aforementioned composition in the manufacture of a medicament for preventing and/or treating a CD20-expressing cancer or immune disease.

In some embodiments, the CD20-expressing cancer is lymphoma or leukemia.

In some embodiments, the lymphoma is non-Hodgkin's lymphoma, B-cell non-Hodgkin's lymphoma, follicular non-Hodgkin's lymphoma, small lymphocytic lymphoma, or diffuse large B-cell lymphoma.

In some embodiments, the leukemia is chronic lymphocytic leukemia, hairy cell leukemia, B-cell prolymphocytic leukemia, or acute lymphocytic leukemia.

In some embodiments, the CD20-expressing immune disease is rheumatoid arthritis, granulomatosis with polyangiitis, Wegener's granulomatosis, microscopic polyangiitis, or multiple sclerosis.

In some embodiments, the rheumatoid arthritis is a severely active rheumatoid arthritis that has failed treatment with at least one TNF antagonist.

Preferably, in some embodiments, the CD20-expressing cancer is CD20-positive B-cell lymphoma.

In some embodiments, the CD20-positive B-cell lymphoma is anti-CD20 monoclonal antibody (e.g., rituximab)-resistant CD20-positive B-cell lymphoma.

In some embodiments, the lymphoma is non-Hodgkin's lymphoma.

In some embodiments, the lymphoma is anti-CD20 monoclonal antibody (e.g., rituximab)-resistant non-Hodgkin's lymphoma.

In some embodiments, the non-Hodgkin's lymphoma is diffuse large B-cell lymphoma.

In some embodiments, the non-Hodgkin's lymphoma is anti-CD20 monoclonal antibody (e.g., rituximab)-resistant diffuse large B-cell lymphoma.

In the sixth aspect of the present invention, the present invention provides a method for preventing and/or treating a CD20-expressing cancer or immune disease, comprising administering to a subject in need thereof a prophylactically and/or therapeutically effective amount of the aforementioned antibody-drug conjugate, or the aforementioned antibody-drug conjugate preparation, or the antibody-drug conjugate preparation prepared by the aforementioned method, or the aforementioned composition.

In some embodiments, the CD20-expressing cancer is lymphoma or leukemia.

In some embodiments, the lymphoma is non-Hodgkin's lymphoma, B-cell non-Hodgkin's lymphoma, follicular non-Hodgkin's lymphoma, small lymphocytic lymphoma, or diffuse large B-cell lymphoma.

In some embodiments, the leukemia is chronic lymphocytic leukemia, hairy cell leukemia, B-cell prolymphocytic leukemia, or acute lymphocytic leukemia.

In some embodiments, the CD20-expressing immune disease is rheumatoid arthritis, granulomatosis with polyangiitis, Wegener's granulomatosis, microscopic polyangiitis, or multiple sclerosis.

In some embodiments, the rheumatoid arthritis is a severely active rheumatoid arthritis that has failed treatment with at least one TNF antagonist.

Preferably, in some embodiments, the CD20-expressing cancer is CD20-positive B-cell lymphoma.

In some embodiments, the CD20-positive B-cell lymphoma is anti-CD20 monoclonal antibody (e.g., rituximab)-resistant CD20-positive B-cell lymphoma.

In some embodiments, the lymphoma is non-Hodgkin's lymphoma.

In some embodiments, the lymphoma is anti-CD20 monoclonal antibody (e.g., rituximab)-resistant non-Hodgkin's lymphoma.

In some embodiments, the non-Hodgkin's lymphoma is diffuse large B-cell lymphoma.

In some embodiments, the non-Hodgkin's lymphoma is anti-CD20 monoclonal antibody (e.g., rituximab)-resistant diffuse large B-cell lymphoma.

In some embodiments, when the subject is an NHL PDX model, the dose administered to the subject in need thereof is 0.3 to 10 mg/kg (e.g., 0.3 mg/kg, 1 mg/kg, 3 mg/kg, 10 mg/kg).

In some embodiments, when the subject is an NHL PDX model, the dose administered to the subject in need thereof is 0.3 to 3 mg/kg, 1 to 3 mg/kg, 1 to 10 mg/kg, or 3 to 10 mg/kg.

In some embodiments, when the subject is a human, the dose administered to the subject in need thereof is

0.1 to 5 mg/kg (e.g., 0.1 mg/kg, 0.3 mg/kg, 1 mg/kg, 3 mg/kg or 5 mg/kg).

In some embodiments, a prophylactically and/or therapeutically effective amount of the aforementioned antibody-drug conjugate, or the aforementioned antibody-drug conjugate preparation, or the antibody-drug conjugate preparation prepared by the aforementioned method, or the aforementioned composition, is administered to the subject in need thereof once every 1, 2, 3, 4, 5, or 6 weeks.

In some embodiments, a prophylactically and/or therapeutically effective amount of the aforementioned antibody-drug conjugate, or the aforementioned antibody-drug conjugate preparation, or the antibody-drug conjugate preparation prepared by the aforementioned method, or the aforementioned composition, is administered to the subject in need thereof once every 18 to 24 days.

In some embodiments, the method further comprises administering an additional chemotherapeutic drug or immunosuppressive agent for treatment of tumor to the subject in need thereof.

In some embodiments, the chemotherapeutic drug is, for example, doxorubicin (Adriamycin), cyclophosphamide and taxanes [paclitaxel (Taxol) and docetaxel (Taxotere)], capecitabine (Xeloda), gemcitabine (Gemzar), vinorelbine (Navelbine), tamoxifen, aromatase inhibitor (Arimidex, Femara, Aromasin), 5-FU/leucovorin, irinotecan (camptosar), oxaliplatin, cisplatin, carboplatin, estramustine, mitoxantrone (Novantrone), prednisone, vincristine (Oncovin), Doxorubicin, prednisone, etc., or a combination thereof.

In some embodiments, the immunosuppressive agent is selected from: (1) glucocorticoid, such as cortisone and prednisone; (2) microbial metabolite, such as cyclosporine and tacrolimus, etc.; (3) anti-metabolite, such as azathioprine and 6-mercaptopurine, etc.; (4) polyclonal and monoclonal anti-lymphocyte antibodies, such as anti-lymphocyte globulin and OKT3, etc.; (5) alkylating agent, such as cyclophosphamide. Specifically, the immunosuppressive agent is, for example, methylprednisolone, prednisone, azathioprine, Prograf, Zenapax, Simulect, cyclosporine, tacrolimus, rapamycin, mycophenolate, mizoribine, cyclophosphamide, Fingolimod, etc.

In the seventh aspect of the present invention, the present invention provides the aforementioned antibody-drug conjugate, or the aforementioned antibody-drug conjugate preparation, or the antibody-drug conjugate preparation prepared by the aforementioned method, or the aforementioned composition, for use in preventing and/or treating a CD20-expressing cancer or immune disease.

In some embodiments, the CD20-expressing cancer is lymphoma or leukemia.

In some embodiments, the lymphoma is non-Hodgkin's lymphoma, B-cell non-Hodgkin's lymphoma, follicular non-Hodgkin's lymphoma, small lymphocytic lymphoma, or diffuse large B-cell lymphoma.

In some embodiments, the leukemia is chronic lymphocytic leukemia, hairy cell leukemia, B-cell prolymphocytic leukemia, or acute lymphocytic leukemia.

In some embodiments, the CD20-expressing immune disease is rheumatoid arthritis, granulomatosis with polyangiitis, Wegener's granulomatosis, microscopic polyangiitis, or multiple sclerosis.

In some embodiments, the rheumatoid arthritis is a severely active rheumatoid arthritis that has failed treatment with at least one TNF antagonist.

Preferably, in some embodiments, the CD20-expressing cancer is CD20-positive B-cell lymphoma.

In some embodiments, the CD20-positive B-cell lymphoma is anti-CD20 monoclonal antibody (e.g., rituximab)-resistant CD20-positive B-cell lymphoma.

In some embodiments, the lymphoma is non-Hodgkin's lymphoma.

In some embodiments, the lymphoma is anti-CD20 monoclonal antibody (e.g., rituximab)-resistant non-Hodgkin's lymphoma.

In some embodiments, the non-Hodgkin's lymphoma is diffuse large B-cell lymphoma.

In some embodiments, the non-Hodgkin's lymphoma is anti-CD20 monoclonal antibody (e.g., rituximab)-resistant diffuse large B-cell lymphoma.

In some embodiments, when the subject is an NHL PDX model, the dose administered is 0.3 to 10 mg/kg (e.g., 0.3 mg/kg, 1 mg/kg, 3 mg/kg, 10 mg/kg).

In some embodiments, when the subject is an NHL PDX model, the dose administered is 0.3 to 3 mg/kg, 1 to 3 mg/kg, 1 to 10 mg/kg, or 3 to 10 mg/kg.

In some embodiments, when the subject is a human, the dose administered is 0.1 to 5 mg/kg (e.g., 0.1 mg/kg, 0.3 mg/kg, 1 mg/kg, 3 mg/kg, or 5 mg/kg).

Beneficial effects:

1. ADC-1 is an ADC formed by coupling Rituximab biosimilar MAB801 with vc-MMAE, and kills CD20-positive non-Hodgkin's lymphoma (NHL) cells by binding to the CD20 receptor on the surface of tumor cells, endocytoses and release of MMAE, thereby controlling tumor growth and causing tumor regression;

2. ADC-1 exhibits significant pharmacodynamic effects of inhibiting tumor cell growth in various CD20-expressing NHL cell lines and various CD20-expressing human NHL PDX tumor models, especially it also exhibits significant

inhibitory effect on tumor cell growth in Rituxan®-resistant NHL PDX model.

3. In the preparation process of ADC-1, the entire reaction process only needs to be completed sequentially in one container without intermediate purification steps, which is superior to other preparation processes.

4. In the preparation process of ADC-1, only one ultrafiltration purification step is required for the whole process, and no chromatography process is required, which is superior to other preparation processes.

5. ADC-1 has good formulation stability.

## Brief Description of the Drawings

Figure 1 shows the HIC-HPLC spectrum of ADC-1 of the example of the present invention;

Figure 2 shows the representative cell-killing effect curve of ADC-1 and Rituxan@ in Daudi cell line according to the example of the present invention, wherein, Inhibition represents inhibition rate;

Figure 3 shows the representative cell-killing effect curve of ADC-1 and Rituxan@ in Jeko-1 cell line according to the example of the present invention, wherein, Inhibition represents inhibition rate;

Figure 4 shows the representative cell-killing effect curve of ADC-1 and Rituxan@ in Raji cell line according to the example of the present invention, wherein, Inhibition represents inhibition rate;

Figure 5 shows the representative cell-killing effect curve of ADC-1 and Rituxan@ in Ramos cell line according to the example of the present invention, wherein, Inhibition represents inhibition rate;

Figure 6 shows the schematic diagram of the effect of ADC-1 and Rituxan® on the tumor volume of CD20-positive human lymphoma (NHL) PDX model LYM#004 according to the example of the present invention;

Figure 7 shows the schematic diagram of the effect of ADC-1 and Rituxan® on the tumor volume of CD20-positive human lymphoma (NHL) PDX model LYM#013 according to the example of the present invention;

Figure 8 shows the schematic diagram of the effect of ADC-1 and Rituxan® on the tumor volume of CD20-positive human lymphoma (NHL) PDX model LYM#016 according to the example of the present invention;

Figure 9 shows the schematic diagram of the effect of pH on the SEC stability of the active substance ADC-1 (10 mM histidine buffer system), wherein ADC-1 is in 10 mM histidine system with different pH, the left figure shows the change trend of SEC monomer content of ADC-1 at 25°C for 6 weeks and at 40°C for 4 weeks, and the right figure shows the change trend of SEC polymer content of ADC-1 at 25°C for 6 weeks and at 40°C for 4 weeks.

## Specific Models for Carrying Out the present invention

The embodiments of the present invention will be described in detail below with reference to the examples, but those skilled in the art will understand that the following examples are only used to illustrate the present invention, and should not be regarded as limiting the scope of the present invention. If a specific condition is not indicated in the examples, it is carried out according to the conventional condition or the condition suggested by the manufacturer. The reagents or instruments used without the manufacturer's indication are conventional products that can be obtained from the market.

In the present invention, unless otherwise specified, scientific and technical terms used herein have the meanings commonly understood by those skilled in the art. Moreover, the protein and nucleic acid chemistry, molecular biology, cell and tissue culture, microbiology, immunology related terms and laboratory procedures used herein are the terms and routine procedures widely used in the corresponding fields. Meanwhile, for a better understanding of the present invention, definitions and explanations of related terms are provided below.

In the present invention, any concentration range, percentage range, ratio range or numerical range should be understood to include any integer value within the stated range and, where appropriate, any fractional value within the range, unless otherwise indicated.

In the present invention, the term "antibody" refers to an immunoglobulin molecule generally composed of two identical pairs of polypeptide chains, each pair having one "light" (L) chain and one "heavy" (H) chain. The light chains of antibodies can be divided into two categories: κ and λ. Heavy chains can be divided into five types: μ, δ, γ, α or ε, and antibodies can be divided into five types: IgM, IgD, IgG, IgA and IgE according to the difference of heavy chain. Within the light and heavy chains, the variable and constant regions are linked by a "J" region of about 12 or more amino acids, and the heavy chain also contains a "D" region of about 3 or more amino acids. Each heavy chain consists of a heavy chain variable region ($V_H$) and a heavy chain constant region ($C_H$). The heavy chain constant region consists of 3 domains ($C_H1$, $C_H2$ and $C_H3$). Each light chain consists of a light chain variable region ($V_L$) and a light chain constant region ($C_L$). The light chain constant region consists of one domain, $C_L$. The constant regions of the antibodies can mediate the binding of immunoglobulin to host tissues or factors, including various cells of the immune system (e.g., effector cells) and component C1q of the complement system. The $V_H$ and $V_L$ regions can also be subdivided into regions of high variability called complementarity determining regions (CDRs) which are interspersed with more conserved regions called framework regions (FRs). Each $V_H$ and $V_L$ consists of 3 CDRs and 4 FRs arranged in the following order: FR1, CDR1, FR2, CDR2, FR3, CDR3, FR4 from amino terminus to carboxy terminus. The variable regions ($V_H$ and $V_L$) of each heavy chain/light chain pair form the antibody binding site respectively. The assignment of amino acids to regions or domains follows the definitions of Kabat Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

The antibody suitable for use in the present invention is an antibody targeting CD20, and the anti-CD20 monoclonal antibody is any antibody targeting CD20, such as rituximab or a biosimilar thereof. Among them, the biosimilar refers to an antibody product with the same sequence as rituximab, the same physicochemical properties and biological activities, and the same clinical safety and efficacy as rituximab.

In the present invention, the structure of MMAE is:

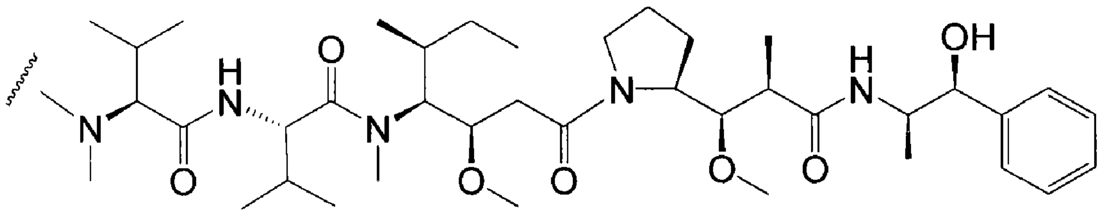

In the present invention, the drug: antibody ratio (DAR) or drug loading (loading) is represented by p or q, that is, the average number of drug modules (i.e. cytotoxic agent) per antibody in the molecule of Formula I: Ab-$(L\text{-}D)_p$ or Formula II: Ab-$(L\text{-}D)_q$, which can be either an integer or a fraction. The ADC of Formula I comprises a collection of antibodies conjugated with a range of (3.6 to 4.0) drug modules, and the ADC of Formula II comprises a collection of antibodies conjugated with a range of (3.3 to 4.3) drug modules. The average number of drug modules per antibody in the ADC preparation from conjugation reaction can be verified by conventional means, such as mass spectrometry, ELISA assay, HIC and HPLC. The quantitative distribution of the ADC in terms of p or q can also be determined. In some cases, the separation, purification and validation of homogeneous ADCs where p or q is a certain value from ADCs with other drug loading can be accomplished by means such as reverse phase HPLC or electrophoresis.

In certain embodiments, the drug module at an amount of less than the theoretical maximum is conjugated to the antibody in the conjugation reaction. In general, the antibody does not comprise many free and reactive cysteine thiol groups that can link drug modules; in fact, most of the cysteine thiol groups in the antibody exist as disulfide bridges. In certain embodiments, the antibody can be reduced with a reducing agent such as dithiothreitol (DTT) or tris(2-carboxyethyl)phosphine (TCEP) under partially or fully reducing conditions to generate reactive cysteine thiol groups.

The antibody-drug conjugate of the present invention can be used in combination with a known chemotherapeutic drug or immunosuppressive agent for the treatment of a tumor, in which the chemotherapeutic drug can be, for example, doxorubicin (Adriamycin), cyclophosphamide and taxanes [paclitaxel (Taxol) and docetaxel (Taxotere)], capecitabine (Xeloda), gemcitabine (Gemzar), vinorelbine (Navelbine), tamoxifen, aromatase inhibitor (Arimidex, Femara, Aromasin), 5-FU/leucovorin, irinotecan (camptosar), oxaliplatin, cisplatin, carboplatin, estramustine, mitoxantrone (Novantrone), prednisone, vincristine (Oncovin), Doxorubicin, prednisone, etc., or a combination thereof; and the immunosuppressive agent is selected from: (1) glucocorticoid, such as cortisone and prednisone; (2) microbial metabolite, such as cyclosporine and tacrolimus, etc.; (3) anti-metabolite, such as azathioprine and 6-mercaptopurine, etc.; (4) polyclonal and monoclonal anti-lymphocyte antibodies, such as anti-lymphocyte globulin and OKT3, etc.; (5) alkylating agent, such as cyclophosphamide. Specifically, the immunosuppressive agent is, for example, methylprednisolone, prednisone, azathioprine, Prograf, Zenapax, Simulect, cyclosporine, tacrolimus, rapamycin, mycophenolate, mizoribine, cyclophosphamide, Fingolimod, etc.

In the present invention, "treatment" refers to a clinical intervention that attempts to alter the natural course of the individual or cells being treated, either for prophylaxis or in the course of clinical pathology. Desired effects of treatment include preventing the occurrence or recurrence of disease, alleviating symptom, attenuating any direct or indirect pathological consequences of disease, preventing metastasis, slowing the rate of disease progression, ameliorating or alleviating the disease state, and relieving or improving prognosis. In some embodiments, the antibody or antibody-drug conjugate of the present invention is used to delay the onset of a disease or disorder, or to slow the progression of a disease or disorder. The above parameters used to assess successful treatment and improvement of disease can be readily measured by routine procedures familiar to physicians. For cancer therapy, efficacy can be measured, for example, by assessing time to progression (TTP) of disease and/or determining response rate (RR).

In the present invention, "subject" refers to a vertebrate. In certain embodiments, the vertebrate refers to a mammal. The mammal includes, but is not limited to, livestock (e.g., cattle), pet (e.g., cat, dog, and horse), primate, mouse, and rat. In certain embodiments, the mammal refers to a human.

In the present invention, an "effective amount" refers to an amount effective at the necessary dose and time to achieve the desired therapeutic or prophylactic effect. A "therapeutically effective amount" of the substance/molecule of the present invention may vary depending on factors such as the disease state, the age, sex and weight of the individual and the ability of the substance/molecule to elicit a desired response in the individual. A therapeutically effective amount also encompasses an amount in which any toxic or detrimental consequences of the substance/molecule are outweighed by the therapeutically beneficial effects. A "prophylactically effective amount" refers to an amount effective at the necessary dose and time to achieve the desired prophylactic effect. Usually, but not necessarily, the prophylactically effective amount will be less than the therapeutically effective amount because the prophylactic dose is administered to the subject prior to the onset of the disease or at an early stage of the disease. In the case of cancer, a therapeutically effective amount of the drug can reduce the number of cancer cells; shrink the tumor size; inhibit (i.e., slow to some extent, preferably stop) the infiltration of cancer cells into surrounding organs; inhibit (i.e., slow to some extent, preferably stop) the tumor metastasis; inhibit to some extent the tumor growth; and/or reduce to some extent one or more symptoms associated with cancer.

For the prevention or treatment of disease, the appropriate dose of the antibody-drug conjugate of the present invention (when used alone or in combination with one or more additional therapeutic agents such as chemotherapeutic agent) will depend on the type of disease to be treated, type of antibody-drug conjugate, severity and course of disease, whether the antibody-drug conjugate is administered for prophylactic or therapeutic purposes, previous therapy, patient's clinical history and reactivity to the antibody-drug conjugate, and attending physician's judgment. It is suitable that the antibody-drug conjugate is administered to the patient at one time or over a series of treatments. Exemplary dose of the antibody-drug conjugate may range from about 0.1 mg/kg to about 5 mg/kg. As such, one or more doses of about 0.1 mg/kg, 0.3 mg/kg, 1.0 mg/kg, 3.0 mg/kg, or 5 mg/kg (or any combination thereof) of the antibody-drug conjugate can be administered to the patient.

"Pharmaceutically acceptable carrier" as used in the present invention includes pharmaceutically acceptable carrier, excipient or stabilizer, which are non-toxic at the dosages and concentrations employed to the cells or mammals to which they are exposed. Typically, the physiologically acceptable carrier is a pH buffered aqueous solution. Examples of physiologically acceptable carrier include buffer, such as phosphate, citrate, and other organic acid; antioxidant, including ascorbic acid; low molecular weight (less than about 10 residues) polypeptide; protein, such as serum albumin, gelatin, or immunoglobulin; hydrophilic polymer, such as polyvinylpyrrolidone; amino acid, such as glycine, glutamine, asparagine, arginine, or lysine; monosaccharide, disaccharide, and other carbohydrate, including glucose, mannose, sucrose, trehalose or dextrin; chelating agent, such as EDTA; sugar alcohol, such as mannitol or sorbitol; salt-forming counterion, such as sodium; and/or nonionic surfactant, such as TWEEN™, polyethylene glycol (PEG) and PLURONICS™.

In the present invention, the 20 conventional amino acids and their abbreviations follow the conventional usage. See, Immunology-A Synthesis (2nd Edition, E.S. Golub and D.R. Gren, Eds., Sinauer Associates, Sunderland, Mass. (1991)), which is incorporated herein by reference.

The present invention will be further explained below in conjunction with specific examples. Example 1: Construction of production cell line for antibody MAB801 (used for the naked antibody moiety of antibody-drug conjugate ADC-1)

The marketed drug Mabthera (Rituxan®, Rituximab) was analyzed by LC/MS/MS, and the amino acid sequences of the light and heavy chains of the antibody were determined. The full gene sequences of the light chain and heavy chain were further confirmed according to the sequence information of the patent (Anderson-1998, patent number: US5736137A), the light chain and heavy chain gene DNA sequences of MAB801 were obtained through gene synthesis, respectively, and then the light chain (Light Chain, LC) and heavy chain (Heavy Chain, HC) gene expression plasmids were constructed separately using a dual-plasmid vector expression system (from Invitrogen Company), the two plasmids were co-transfected into mammalian host cell CHO DG44 (from Invitrogen Company), and after two-step stress screening, a cell population simultaneously integrated with the light chain and heavy chain gene sequences was obtained. The

antibody expression ability of the cell population was evaluated and found to meet the requirements. The single-cell clones were screened using semi-solid medium, and the obtained single-cell clones were screened by fed-batch experiments to select the cloned cell line with the highest expression level of antibody, and on the basis of this cell line, a production cell bank of MAB 801 was prepared.

Gene sequencing was performed on the monoclonal cell line, and the sequencing results showed that the sequences encoding the light chain and heavy chain of MAB801 were completely consistent with the commercially available rituximab.

Example 2: Preparation of antibody MAB801 (for the naked antibody moiety of antibody-drug conjugate ADC-1)

MAB801 was prepared by the fed-batch cell culture process widely used in the antibody field. This process, starting from the recovery of cell seeds of the working cell bank, comprised the main steps of: shaking flask inoculation, shaking flask cell expansion, cell expansion culture step by step in the reactor, and then inoculation into the production reactor for fed-batch culture, followed by purification processes comprising: fermentation broth clarification (depth filtration), Protein A affinity chromatography, low pH virus inactivation and depth filtration, PB incubation, anion exchange chromatography, cation exchange chromatography, virus removal filtration, as well as tangential flow ultrafiltration and diafiltration and other steps, and then filtration and packaging for obtaining the product.

The detection results showed that the produced rituximab biosimilar was highly similar to rituximab in terms of molecular weight and other physical and chemical indicators.

Example 3: Preparation method of ADC-1, CD20 targeting antibody-drug conjugate

1) 10 mg of MAB801 antibody was taken and subjected to replacement into a reducing buffer (25 mM sodium borate, pH 8.0, 25 mM NaCl, 5 mM EDTA) by using a 15 mL 30KD ultrafiltration device, and a total of three replacements were performed; the final volume was approximately 1 mL, the obtained solution was transferred to a new Eppendorf centrifuge tube (weighed), and weighed; the protein concentration was detected, and the total protein was calculated.

2) DTT at 2.5 times moles was added to the antibody, incubated at room temperature for 2 hours, and mixed continuously; the obtained solution was subjected to replacement into a coupling buffer (50 mM Tris, pH 7.2, 150 mM NaCl, 5 mM EDTA) by using a 15mL 30KD ultrafiltration device, a total of three replacements were performed. The concentrated solution was taken, the protein concentration was determined with $A_{280}$, and weighing was performed to calculate the total amount of protein; a 10μL sample was taken and the number of free sulfhydryl groups was determined by Ellman's method;

   and the molar concentration of the free sulfhydryl groups was calculated with the following formula:

$$C_{thiol} = \frac{A412 \times 11.2}{b \times 14150} \ (M)$$

   b: optical path length of cuvette (usually 1 cm)

   the number of moles of free sulfhydryl groups was calculated based on the molar concentration of free sulfhydryl groups and the total protein solution volume.

3) vc-MMAE (dissolved in DMSO) was added at 1.1 times the moles of free sulfhydryl groups to the reduced antibody, mixed well, and then reacted at room temperature for 2 hours with intermittent agitation. To the reaction system, N-acetylcysteine was added at 20 times the moles of vc-MMAE into the reaction solution, mixed well, and allowed to stand for 5 minutes.

4) the obtained solution in step 3) was subjected to replacement into a conjugate stock solution (10 mM histidine, 6% sucrose, 0.035% PS80, pH 5.8) by using a 15 mL 30KD ultrafiltration device, and a total of three replacements were performed to obtain the antibody-drug conjugate ADC-1, which was stored at 4°C, wherein the concentration of the active ingredient antibody-drug conjugate was 5 mg/mL.

Example 4: Determination of drug/antibody ratio in ADC-1

The prepared antibody-drug conjugate ADC-1 was analyzed by HIC-HPLC (Ouyang-2013) to determine the drug/antibody ratio (DAR), the results were shown in Figure 1, in which the average drug loading DAR was 3.8 according to the calculation of peak area of the spectrum.

Example 5: In vitro killing activity of ADC-1 on CD20-expressing NHL cells

In order to study the killing effect of ADC-1 on CD20-expressing NHL cells, four kinds of CD20-expressing NHL cell lines, Daudi, Jeko-1, Raji and Ramos, were selected, and the cell proliferation inhibition method using PrestoBlue® detection reagent was adopted to determine the killing effects of ADC-1 and the commercial reference drug Rituxan@ on the above four cell lines, and the results were shown in Table 2, as well as Figure 2, Figure 3, Figure 4 and Figure 5.

Table 2: Killing effects of ADC-1 and Rituxan® on four CD20-expressing NHL cell lines

| Cell line | Tissue source | CD20 expression level | $IC_{50}$ (Mean±SD, ng/mL) | |
|---|---|---|---|---|
| | | | ADC-1 | Rituxan® |
| Daudi | Lymphoma | High expression | 155.4±43.8 | ND |
| Jeko-1 | Lymphoma | High expression | 23.1±4.4 | ND |
| Raji | Lymphoma | High expression | 140.8±26.4 | ND |
| Ramos | Lymphoma | High expression | 39.6±8.9 | ND |
| Note: References for CD20 expression levels were Barth-2015, Law-2004. $IC_{50}$ value was the average value of $IC_{50}$ calculated for two 96-well plates; ND indicated that the highest percentage of inhibition of proliferation (inhibition%) was less than 50%, so that $IC_{50}$ value could not be calculated. | | | | |

The experimental results showed that the ADC-1 had a significant cell-killing effect on the four CD20-expressing NHL cell lines, and the effect was significantly better than that of the commercial reference drug Rituxan@.

Example 6: Antitumor effect of ADC-1 in NHL PDX model

Human-derived tumor tissue xenograft model (PDX model) is a tumor model established in immunodeficient mice using human primary tumor tissue, which preserves the heterogeneity, molecular diversity and histology characteristics of primary tumors to the greatest extent. It has high predictive value for the clinical treatment effect of drugs, and has been increasingly used in cancer research in recent years (Hidalgo to 2014). To effectively evaluate the efficacy of ADC-1 in future clinical indications, experiments were performed in three CD20-expressing human lymphoma cell carcinoma (NHL) PDX models. In all experiments, the commercially available CD20-targeted monoclonal antibody Rituxan® was used as a reference drug to compare tumor-suppressive activity. Of these three NHL PDX models, two were resistant to Rituxan®.

The information of the three PDX models used in the study was shown in Table 3.

Table 3: PDX model information used for in vivo pharmacodynamic experiment

| Model No. | Sex | Age | Pathological diagnosis | Passage number used in experiment | CD20 IHC | Rituxan® -resistant |
|---|---|---|---|---|---|---|
| LYM#004 | Female | 53 | Diffuse large B-cell lymphoma (non-GCB type) | P7 | Positive | Yes |
| LYM#013 | Female | 13 | (Mesenteric) Diffuse Large B-cell Lymphoma (GCB) | P6 | Positive | Yes |
| LYM#016 | Female | 83 | Peripheral B-cell lymphoma-diffuse large B-cell lymphoma (GCB) | P6 | Positive | no |

1. Study of efficacy of ADC-1 in CD20-positive NHL PDX model LYM#004

LYM#004 was a Rituxan®-resistant DLBCL PDX model, and the experimental results were shown in Figure 6. After ADC-1 was administered at doses of 1, 3 and 10 mg/kg, on Day 18, T/C (%) was 45.90% (P>0.05), 4.46% (P<0.001) and 2.05% (P<0.001), respectively; and TGI% was 54.10%, 95.54% and 97.95%, respectively. After ADC-1 was administered at dose of 3 mg/kg, all tumors partially regressed on Day 18; after ADC-1 was administered at dose of 10 mg/kg, 5/8 tumors partially regressed and 3/8 tumors completely regressed on Day 18, respectively. After administration of Rituxan® at doses of 3 and 10 mg/kg, on Day 18, T/C (%) was 50.76% (P>0.05) and 51.50% (P>0.05), respectively; and TGI% was 49.24% and 48.50%, respectively.

The experimental results showed that ADC-1 (3 and 10 mg/kg) had significant inhibitory activity against tumor growth, while ADC-1 (1 mg/kg) and the reference drug Rituxan® (3 and 10 mg/kg) had no significant anti-tumor activity. Both ADC-1 and Rituxan® were well tolerated by tumor-bearing mice.

2. Study of efficacy of ADC-1 in CD20-positive NHL PDX model LYM#013

LYM#013 was a Rituxan®-resistant DLBCL PDX model, and the experimental results were shown in Figure 7. After ADC-1 was administered at doses of 0.3, 1 and 3 mg/kg, on Day 28, T/C (%) was 31.90% (P<0.05), 0.00% (P<0.001) and 0.00% (P<0.001), respectively; TGI% was 68.10%, 100.00% and 100.00%, respectively; 2/8, 0/8 and 0/8 tumors partially regressed, and 0/8, 8/8 and 8/8 tumors completely regressed, respectively. After Rituxan@ was administered at dose of 3 mg/kg, on Day 28, T/C (%) was 79.86% (P>0.05), and TGI% was 20.14%.

The experimental results showed that ADC-1 (0.3, 1 and 3 mg/kg) could significantly inhibit tumor growth in a dose-dependent manner, while the reference drug Rituxan@ (3 mg/kg) had no significant anti-tumor activity. Both ADC-1 and Rituxan@ were well tolerated by tumor-bearing mice.

3. Study of efficacy of ADC-1 in CD20-positive NHL PDX model LYM#016

LYM#016 was a DLBCL PDX model, and the experimental results were shown in Figure 8. After ADC-1 was administered at doses of 0.3, 1 and 3 mg/kg, on Day 14, T/C (%) was 37.76% (P>0.05), 4.27% (P<0.01) and 1.91% (P<0.01), respectively; and TGI% was 62.24%, 95.73% and 98.09%, respectively. After ADC-1 was administered at dose of 1 mg/kg, all tumors partially regressed on Day 14; after ADC-1 was administered at dose of 3 mg/kg, 6/8 tumors partially regressed and 2/8 tumors completely regressed on Day 14, respectively. After Rituxan@ was administered at dose of 3 mg/kg, on Day 14, T/C (%) was 30.98% (P>0.05), and TGI% was 69.02%.

The experimental results showed that ADC-1 (1 and 3 mg/kg) had a significant inhibitory effect on tumor growth, while ADC-1 (0.3 mg/kg) and the reference drug Rituxan@ (3 mg/kg) had no significant anti-tumor activity. Both ADC-1 and Rituxan@ were well tolerated by tumor-bearing mice.

The in vivo efficacy experimental results of ADC-1 in three CD20-positive human-derived lymphoma (NHL) PDX models were shown in Table 4.

Table 4: In vivo efficacy results of ADC-1 (i.v., q4d×4) in CD20-positive human lymphoma PDX models

| Model | Substance to be tested | Dose (mg/kg) | Statistical date | Relative tumor proliferation rate T/C(%) | Inhibition rate of tumor growth TGI% | Tumor regression | | Anti-tumor activity |
|---|---|---|---|---|---|---|---|---|
| | | | | | | Partial regression | Complete regression | |
| LYM#004 | Vehicle (ADC-1 vehicle) | / | Day 18 | 100 | 0 | / | / | / |
| | ADC-1 | 1 | | 45.90 (P>0.05) | 54.10 | / | / | ++++ |
| | | 3 | | 4.46 (P<0.001) | 95.54 | 8/8 | 0/8 | |
| | | 10 | | 2.05 (P<0.001) | 97.95 | 5/8 | 3/8 | |
| | Rituxan® | 3 | | 50.76 (P>0.05) | 49.24 | / | / | + |
| | | 10 | | 51.50 (P>0.05) | 48.50 | / | / | |
| LYM#013 | Vehicle (ADC-1 vehicle) | / | Day 28 | 100 | 0 | / | / | / |
| | ADC-1 | 0.3 | | 31.90 (P<0.05) | 68.10 | 2/8 | 0/8 | ++++ |
| | | 1 | | 0 (P<0.001) | 100.00 | 0/8 | 8/8 | |
| | | 3 | | 0 (P<0.001) | 100.00 | 0/8 | 8/8 | |
| | Rituxan® | 3 | | 79.86 (P>0.05) | 20.14 | / | / | + |
| LYM#016 | Vehicle (ADC-1 vehicle) | / | Day 14 | 100 | 0 | / | / | / |
| | ADC-1 | 0.3 | | 37.76 (P>0.05) | 62.24 | / | / | ++++ |
| | | 1 | | 4.27 (P<0.01) | 95.73 | 8/8 | 0/8 | |

| | | | | | | | | |
|---|---|---|---|---|---|---|---|---|
| | | 3 | | 1.91 (P<0.01) | 98.09 | 6/8 | 2/8 | |
| | Rituxan® | 3 | | 30.98 (P>0.05) | 69.02 | / | / | ++ |

Note: "/" represented "not available". The formula for calculating tumor volume (TV) was: $TV=l\times w^2/2$, wherein l and w represented the measured length and width of tumor, respectively. The relative tumor volume (RTV) was calculated according to the measurement results, $RTV=V_f/V_0$, wherein $V_0$ represented the tumor volume measured at the time of grouping and administration (i.e., Day 0), and $V_f$ represented the tumor volume measured on the last day. Relative tumor proliferation rate T/C(%)=(RTV of administration group/RTV of vehicle group)×100%. Tumor growth inhibition rate TGI%=(average tumor volume of vehicle group - average tumor volume of administration group)/average tumor volume of vehicle group × 100%. "++++" represented T/C (%) ≥0 and ≤10%, "+++" represented T/C (%)>10% and ≤20%, "++" represented T/C (%) >20% and ≤40%, "+" represented T/C(%)>40%. The division of the antitumor activity of ADC-1 and Rituxan® was based on the T/C (%) value at the highest dose in the model.

In conclusion, the results of the in vivo efficacy experiment showed that ADC-1 exhibited significant tumor growth inhibitory effect in three human lymphoma (NHL) PDX models (all models were DLBCL), and very good tolerance in tumor-bearing mice. It was worth pointing out that two of these three DLBCL PDX models were resistant to Rituxan@.

Example 7: Effect of pH on stability of ADC-1 preparation

On the basis of the research on the preparation of ADC-1 and previous experiences, the inventors of the present application finally determined that the preparation was: 5 mg/mL antibody-drug conjugate (naked antibody-drug conjugate without buffer and other components), 10 mM histidine buffer (pH 5.8), 6% sucrose, and 0.035% Tween-80 (PS80).

In order to investigate the effect of different pH in 10 mM histidine buffer on the quality of ADC-1, the solution of ADC-1 was changed into the formulations F1 to F4 (pH: 5.8 to 7.0) to be investigated, and allowed to stand at 25±2°C/60%±5%RH and 40±2°C/75%±5%RH for investigation for 6 weeks and 4 weeks, respectively, and the samples were taken for analysis at the set time points. The detection items included appearance, pH, protein concentration, SEC, iCIEF and HIC. If the sample had obvious opalescence, subsequent related analysis would not be performed. The specific experimental design was shown in Table 5.

Table 5: Experimental design for effects of pH (5.8 to 7.0) on stability of ADC-1 (10mM histidine buffer)

| Formulation | pH | T0 | 25±2°C/60%±5%RH | | 40±2°C/75%±5%RH | | |
|---|---|---|---|---|---|---|---|
| | | | 2W | 6W | 1W | 2W | 4W |
| F1 | 5.8 | √ | √ | √ | √ | √ | √ |
| F2 | 6.2 | √ | √ | √ | √ | √ | √ |
| F3 | 6.7 | √ | √ | √ | √ | √ | √ |
| F4 | 7.0 | √ | √ | √ | √ | √ | √ |
| Note: W represented week, that was, 2W represented 2 weeks; V represented detected. | | | | | | | |

The experimental results showed that when ADC-1 was in 10 mM histidine buffer, its stability decreased with the increase of pH.

After being allowed to stand at 25°C for 6 weeks, all samples were colorless and clear solutions with no change in pH and concentration; the purity of iCIEF decreased, the content of acidic peak increased, and the change speed increased with the increase of pH; the purity of SEC showed a decreasing trend, the content of polymer (HMW) increased, and the change speed increased with the increase of pH; the DAR value did not change significantly.

After being allowed to stand at 40°C for 4 weeks, the stability of the samples showed the same trend, but the change extent was aggravated; when the pH was 6.7 and 7.0, the appearance of the samples changed within one week at 40°C, opalescence appeared, and the degree of opalescence increased with the increase of pH, the UV detection results were distorted due to the presence of particulate matter (opalescence), resulting in the calculated protein content deviating from the actual value; the SEC purity decreased, the contents of polymer and low molecular weight fragment (LMW) increased, and the change speed increased with the increase of pH; the purity of iCIEF decreased, the content of acidic peak increased, the content of basic peak decreased, and the change speed increased with the increase of pH.

The trend diagram of the SEC purity of the samples as a function of pH was shown in Figure 9.

Although specific embodiments of the present invention have been described in detail, those skilled in the art will appreciate that, in light of all the teachings disclosed, various modifications and substitutions of those details may be made, and these changes are within the scope of the present invention. The full scope of the present invention is given by the appended claims and any equivalents thereof.

## Claims

**1.** An antibody-drug conjugate, the antibody-drug conjugate having a structure shown in Formula I,

$$Ab\text{-}(L\text{-}D)_p \qquad \text{Formula I}$$

wherein:

Ab represents an anti-CD20 monoclonal antibody, and the anti-CD20 monoclonal antibody is any antibody targeting CD20, such as rituximab or a biosimilar thereof;
D represents a cytotoxic agent, and the cytotoxic agent is Monomethyl auristatin E (MMAE);
L represents a linker for linking the anti-CD20 monoclonal antibody with the cytotoxic agent, and the linker is 6-maleimidocaproyl-valine-citrulline-p-aminobenzyloxycarbonyl (MC-vc-PAB);
p is 3.6 to 4.0, preferably 3.7 to 3.9, more preferably 3.8.

**2.** An antibody-drug conjugate preparation, which comprises:

an antibody-drug conjugate having a concentration of 1 to 60 mg/mL (e.g., a concentration of 1 to 20 mg/mL, or 1 to 10 mg/mL, or 3 to 7 mg/mL, or 5 mg/mL);
a histidine buffer having a concentration of 5 to 35 mM (e.g., a histidine buffer having a concentration of 5 to 15 mM, or 8 to 12 mM, or 10 mM), and a pH of 5.0 to 6.2 (e.g., pH of 5.8 to 6.2, or pH of 5.4 to 6.0, or pH of 5.4 to 6.2, or pH of 5.6 to 6.0);
sucrose having a concentration of 2% to 10% (e.g., sucrose having a concentration of 3% to 9%, or 4% to 8%, or 5% to 7%, or 6%); and
Tween-80 having a concentration of 0.01% to 0.1% (e.g., Tween-80 having a concentration of 0.02% to 0.06%, 0.02% to 0.05%, 0.03% to 0.04% or 0.035%);
the antibody-drug conjugate has a structure shown in Formula II,

$$Ab\text{-}(L\text{-}D)_q \qquad \text{Formula II}$$

wherein:

Ab represents an anti-CD20 monoclonal antibody, and the anti-CD20 monoclonal antibody is any antibody targeting CD20, such as rituximab or a biosimilar thereof;
D represents a cytotoxic agent, and the cytotoxic agent is Monomethyl auristatin E (MMAE);
L represents a linker for linking the anti-CD20 monoclonal antibody with the cytotoxic agent, and the linker is 6-maleimidocaproyl-valine-citrulline-p-aminobenzyloxycarbonyl (MC-vc-PAB);
q is 3.3 to 4.3 (e.g., 3.6 to 4.0, or 3.7 to 3.9, or 3.8);
preferably, the preparation comprises:

the antibody-drug conjugate, having a concentration of 5 mg/mL;
10 mM histidine buffer, pH 5.8;
6% sucrose; and
0.035% Tween-80.

**3.** A preparation method of the antibody-drug conjugate preparation according to claim 2, comprising:

Step 1: performing a reduction reaction of an anti-CD20 monoclonal antibody and a reducing agent to obtain a reduced anti-CD20 monoclonal antibody, in which the anti-CD20 monoclonal antibody is any antibody targeting CD20, such as rituximab or its biosimilar;
Step 2: performing a coupling reaction between the reduced anti-CD20 monoclonal antibody and vc-MMAE;
Step 3: quenching the coupling reaction;
Step 4: performing a buffer replacement of the quenched coupling reaction product to obtain the antibody-drug conjugate preparation, in which the antibody-drug conjugate preparation comprising:

1 to 60 mg/mL of the antibody-drug conjugate (e.g., 1 to 20 mg/mL, or 1 to 10 mg/mL, or 3 to 7 mg/mL, or 5 mg/mL of the antibody-drug conjugate),
5 to 35 mM histidine buffer (e.g., 5 to 15 mM, or 8 to 12 mM, or 10 mM histidine buffer), pH 5.0 to 6.2 (e.g., pH 5.8 to 6.2, or pH 5.4 to 6.0, or pH 5.4 to 6.2, or pH 5.6 to 6.0),
2% to 10% sucrose (e.g., 3% to 9%, or 4% to 8%, or 5% to 7%, or 6% sucrose), and
0.01% to 0.1% Tween-80 (e.g., 0.02% to 0.06%, 0.02% to 0.05%, 0.03% to 0.04%, or 0.035% Tween-80).

**4.** The preparation method according to claim 3, wherein the method comprises:

1) 10 mg of the anti-CD20 monoclonal antibody is taken, and subjected to replacement into a reducing buffer (25mM sodium borate, pH 8.0, 25mM NaCl, 5 mM EDTA) by using a 15 mL 30KD ultrafiltration device, and a total of three replacements are performed; a final volume of about 1 mL is obtained; and the obtained solution is transferred to a new Eppendorf centrifuge tube (weighed), and weighed; the protein concentration is detected and the total protein is calculated;
2) DTT is added at 2.0 to 3.0 times moles to the antibody, incubated at room temperature for 2 hours, and mixed continuously; the obtained solution is subjected to replacement into a coupling buffer (50mM Tris, pH 7.2, 150 mM NaCl, 5 mM EDTA) by using a 15 mL 30KD ultrafiltration device, and a total of three replacements are performed; the concentrated solution is taken, its protein concentration is detected by $A_{280}$ (absorbance at 280 nm wavelength), and it is weighed to calculate the total protein; 10 μL of sample is taken, and the number of free sulfhydryl groups is determined by using Ellman's method;

and the molar concentration of its free sulfhydryl groups is calculated by the following formula:

$$C_{thiol} = \frac{A412 \times 11.2}{b \times 14150}\ (M)$$

b: optical path length of cuvette (usually 1cm)
the number of moles of free sulfhydryl groups is calculated according to the molar concentration of free sulfhydryl groups and the volume of the total protein solution;

3) vc-MMAE (dissolved in DMSO) is added at 1.0 to 1.5 times the number of moles of free sulfhydryl groups to the reduced antibody, mixed well, and then reacted at room temperature for 2 hours with intermittent agitation; to the reaction system, N-acetylcysteine is added at 20 times the number of moles of vc-MMAE moles to the reaction solution, mixed well, and allowed to stand for 5 minutes;
4) the obtained solution in step 3) is subjected to replacement into a conjugate stock solution (10 mM histidine, 6% sucrose, 0.035% PS80, pH 5.8) by using a 15 mL 30KD ultrafiltration device, a total of three replacements are performed, and thus the antibody-drug conjugate preparation is obtained, and stored at 4°C;
preferably, the preparation method comprises:

1) 10 mg of the anti-CD20 monoclonal antibody is taken, and subjected to replacement into a reducing buffer (25mM sodium borate, pH 8.0, 25mM NaCl, 5 mM EDTA) by using a 15 mL 30KD ultrafiltration device, and a total of three replacements are performed; a final volume of about 1 mL is obtained, and the obtained solution is transferred to a new Eppendorf centrifuge tube (weighed), and weighed; the protein concentration

is detected and the total protein is calculated;

2) DTT is added at 2.5 times moles to the antibody, incubated at room temperature for 2 hours, and mixed continuously; the obtained solution is subjected to replacement into a coupling buffer (50mM Tris, pH 7.2, 150 mM NaCl, 5 mM EDTA) by using a 15 mL 30KD ultrafiltration device, and a total of three replacements are performed; the concentrated solution is taken, its protein concentration is detected by $A_{280}$, and it is weighed to calculate the total protein; 10 μL of sample is taken, and the number of free sulfhydryl groups is determined by using Ellman's method;

and the molar concentration of its free sulfhydryl groups is calculated by the following formula:

$$C_{thiol} = \frac{A412 \times 11.2}{b \times 14150} \ (M)$$

b: optical path length of cuvette (usually 1cm)

the number of moles of free sulfhydryl groups is calculated according to the molar concentration of free sulfhydryl groups and the volume of the total protein solution;

3) vc-MMAE (dissolved in DMSO) is added at 1.1 times the number of moles of free sulfhydryl groups to the reduced antibody, mixed well, and then reacted at room temperature for 2 hours with intermittent agitation; to the reaction system, N-acetylcysteine is added at 20 times the number of moles of vc-MMAE moles to the reaction solution, mixed well, and allowed to stand for 5 minutes;

4) the obtained solution in step 3) is subjected to replacement into a conjugate stock solution (10 mM histidine, 6% sucrose, 0.035% PS80, pH 5.8) by using a 15 mL 30KD ultrafiltration device, a total of three replacements are performed, and thus the antibody-drug conjugate preparation is obtained, and stored at 4°C, wherein the active ingredient antibody-drug conjugate has a concentration of 5 mg/mL.

**5.** A composition, which comprises the antibody-drug conjugate according to claim 1, or the antibody-drug conjugate preparation according to claim 2, or the antibody-drug conjugate preparation prepared by the method according to any one of claims 3 to 4, optionally, further comprises at least one pharmaceutically acceptable carrier, diluent or excipient.

**6.** Use of the antibody-drug conjugate according to claim 1, or the antibody-drug conjugate preparation according to claim 2, or the antibody-drug conjugate preparation prepared by the method according to any one of claims 3 to 4, or the composition according to claim 5, in the manufacture of a medicament for preventing and/or treating a CD20-expressing cancer or immune disease.

**7.** The use according to claim 6, wherein the CD20-expressing cancer or immune disease is lymphoma (e.g., non-Hodgkin's lymphoma, B-cell non-Hodgkin's lymphoma, follicular non-Hodgkin's lymphoma, small lymphocytic lymphoma, diffuse large B-cell lymphoma), leukemia (e.g., chronic lymphocytic leukemia, hairy cell leukemia, B-cell prolymphocytic leukemia, acute lymphocytic leukemia), rheumatoid arthritis (e.g., severely active rheumatoid arthritis that has failed treatment with at least one TNF antagonist), granulomatosis with polyangiitis, Wegener's granulomatosis, microscopic polyangiitis, or multiple sclerosis;

preferably, the CD20-expressing cancer is CD20-positive B-cell lymphoma, preferably anti-CD20 monoclonal antibody (e.g., rituximab)-resistant CD20-positive B-cell lymphoma,

more preferably, the lymphoma is non-Hodgkin's lymphoma, preferably anti-CD20 monoclonal antibody (e.g., rituximab)-resistant non-Hodgkin's lymphoma,

furthermore preferably, the non-Hodgkin's lymphoma is diffuse large B-cell lymphoma, preferably anti-CD20 monoclonal antibody (e.g., rituximab)-resistant diffuse large B-cell lymphoma.

**8.** A method for preventing and/or treating a CD20-expressing cancer or immune disease, comprising administering to a subject in need a prophylactically and/or therapeutically effective amount of the antibody-drug conjugate according to claim 1, or the antibody-drug conjugate preparation according to claim 2, or the antibody-drug conjugate preparation prepared by the method according to any one of claims 3 to 4, or the composition according to claim 5.

**9.** The method according to claim 8, wherein the CD20-expressing cancer or immune disease is lymphoma (e.g., non-Hodgkin's lymphoma, B-cell non-Hodgkin's lymphoma, follicular non-Hodgkin's lymphoma, small lymphocytic lymphoma, diffuse large B-cell lymphoma), leukemia (e.g., chronic lymphocytic leukemia, hairy cell leukemia, B-cell

prolymphocytic leukemia, acute lymphocytic leukemia), rheumatoid arthritis (e.g., severely active rheumatoid arthritis that has failed treatment with at least one TNF antagonist), granulomatosis with polyangiitis, Wegener's granulomatosis, microscopic polyangiitis, or multiple sclerosis;

preferably, the CD20-expressing cancer is CD20-positive B-cell lymphoma, preferably anti-CD20 monoclonal antibody (e.g., rituximab)-resistant CD20-positive B-cell lymphoma,
more preferably, the lymphoma is non-Hodgkin's lymphoma, preferably anti-CD20 monoclonal antibody (e.g., rituximab)-resistant non-Hodgkin's lymphoma,
furthermore preferably, the non-Hodgkin's lymphoma is diffuse large B-cell lymphoma, preferably anti-CD20 monoclonal antibody (e.g., rituximab)-resistant diffuse large B-cell lymphoma.

**10.** The method according to claim 8, wherein, when the subject is a human, the dose administered to the subject in need thereof is 0.1 to 5 mg/kg (e.g., 0.1 mg/kg, 0.3 mg/kg, 1 mg/kg, 3 mg/kg, 5 mg/kg);
alternatively, when the subject is an NHL PDX model, the dose administered to the subject in need thereof is 0.3 to 10 mg/kg (e.g., 0.3 mg/kg, 1 mg/kg, 3 mg/kg, 10 mg/kg), preferably 0.3 to 3 mg/kg, 1 to 3 mg/kg, 1 to 10 mg/kg, or 3 to 10 mg/kg.

**11.** The antibody-drug conjugate according to claim 1, or the antibody-drug conjugate preparation according to claim 2, or the antibody-drug conjugate preparation prepared by the method according to any one of claims 3 to 4, or the composition according to claim 5, for use in prevention and/or treatment of a CD20-expressing cancer or immune disease.

**12.** The antibody-drug conjugate, or the antibody-drug conjugate preparation, or the composition for use according to claim 11, wherein the CD20-expressing cancer or immune disease is lymphoma (e.g., non-Hodgkin's lymphoma, B-cell non-Hodgkin's lymphoma, follicular non-Hodgkin's lymphoma, small lymphocytic lymphoma, diffuse large B-cell lymphoma), leukemia (e.g., chronic lymphocytic leukemia, hairy cell leukemia, B-cell prolymphocytic leukemia, acute lymphocytic leukemia), rheumatoid arthritis (e.g., severely active rheumatoid arthritis that has failed treatment with at least one TNF antagonist), granulomatosis with polyangiitis, Wegener's granulomatosis, microscopic polyangiitis or multiple sclerosis;

preferably, the CD20-expressing cancer is CD20-positive B-cell lymphoma, preferably anti-CD20 monoclonal antibody (e.g., rituximab)-resistant CD20-positive B-cell lymphoma,
more preferably, the lymphoma is non-Hodgkin's lymphoma, preferably anti-CD20 monoclonal antibody (e.g., rituximab)-resistant non-Hodgkin's lymphoma,
furthermore preferably, the non-Hodgkin's lymphoma is diffuse large B-cell lymphoma, preferably anti-CD20 monoclonal antibody (e.g., rituximab)-resistant diffuse large B-cell lymphoma.

**13.** The antibody-drug conjugate, or the antibody-drug conjugate preparation, or the composition for use according to claim 11, wherein:

when the subject is a human, the dose administered is 0.1 to 5 mg/kg (e.g., 0.1 mg/kg, 0.3 mg/kg, 1 mg/kg, 3 mg/kg, 5 mg/kg);
alternatively, when the subject is an NHL PDX model, the dose administered is 0.3 to 10 mg/kg (e.g., 0.3 mg/kg, 1 mg/kg, 3 mg/kg, 10 mg/kg), preferably 0.3 to 3 mg/kg, 1 to 3 mg/kg, 1 to 10 mg/kg or 3 to 10 mg/kg.

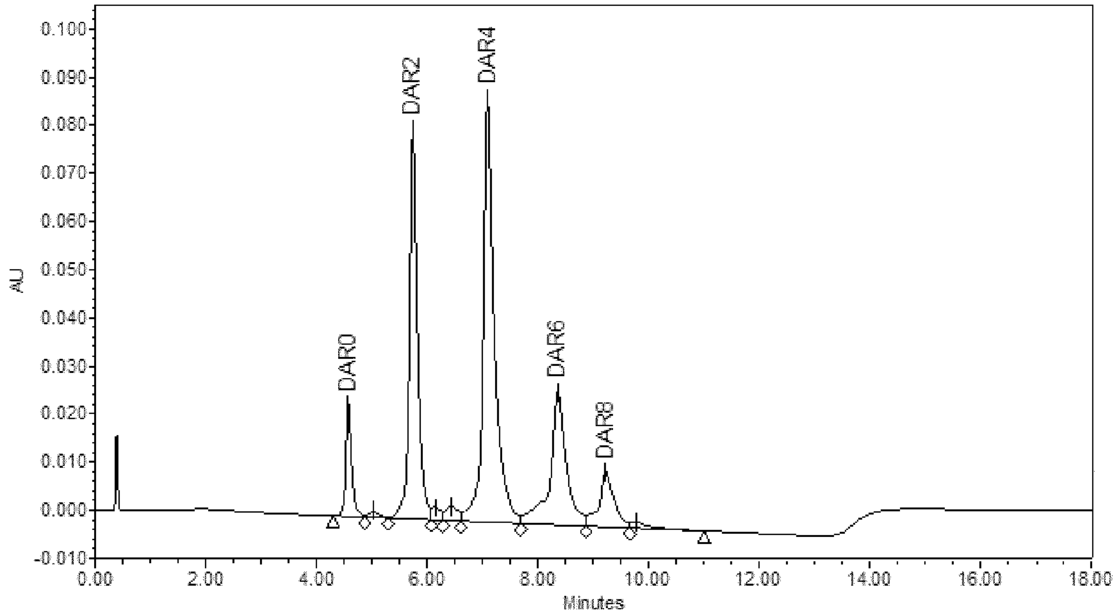


**Figure 1**

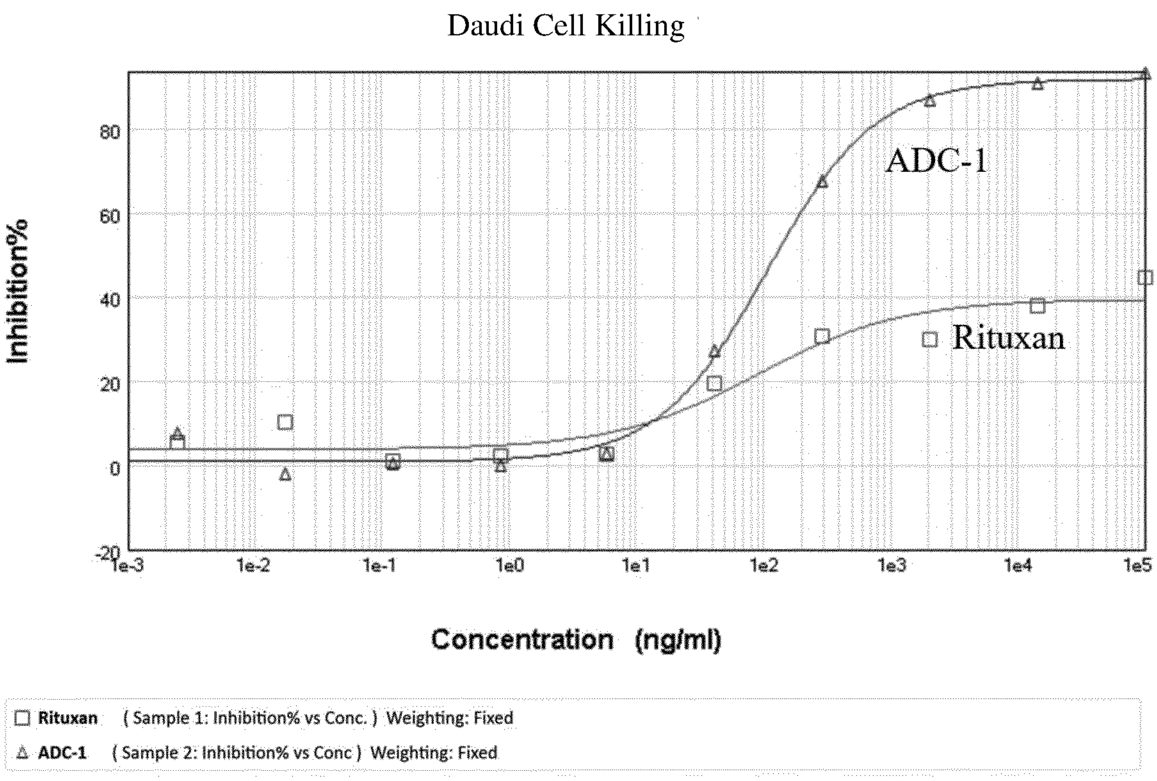


**Figure 2**

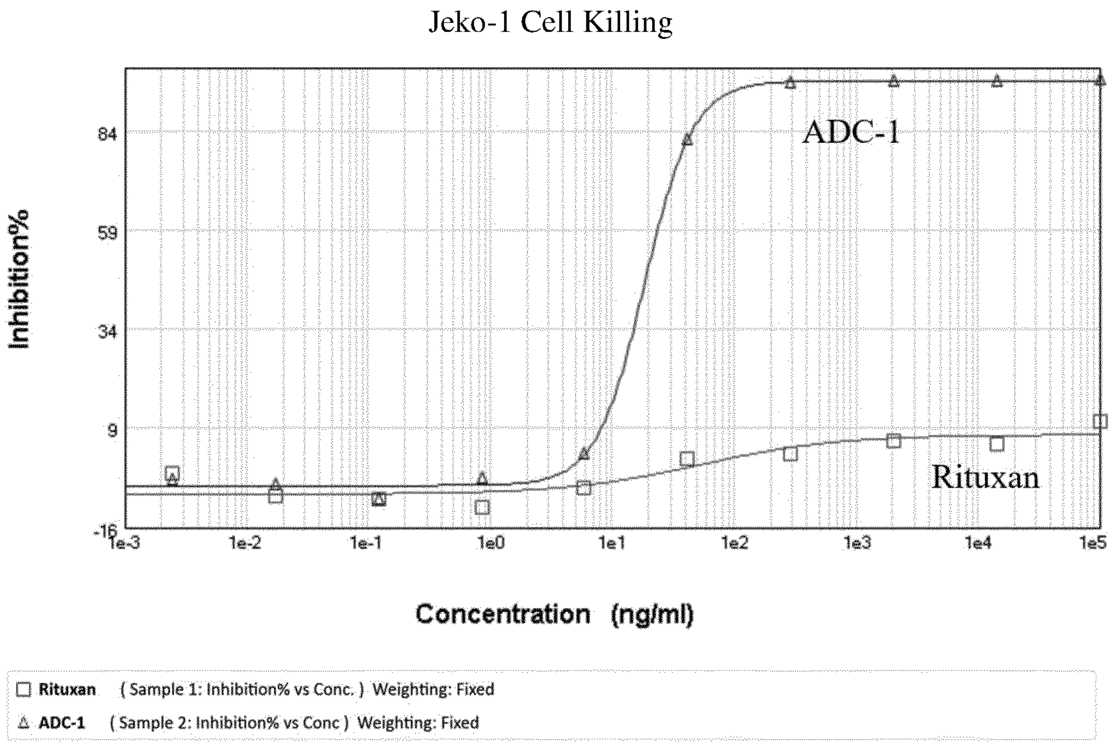


**Figure 3**

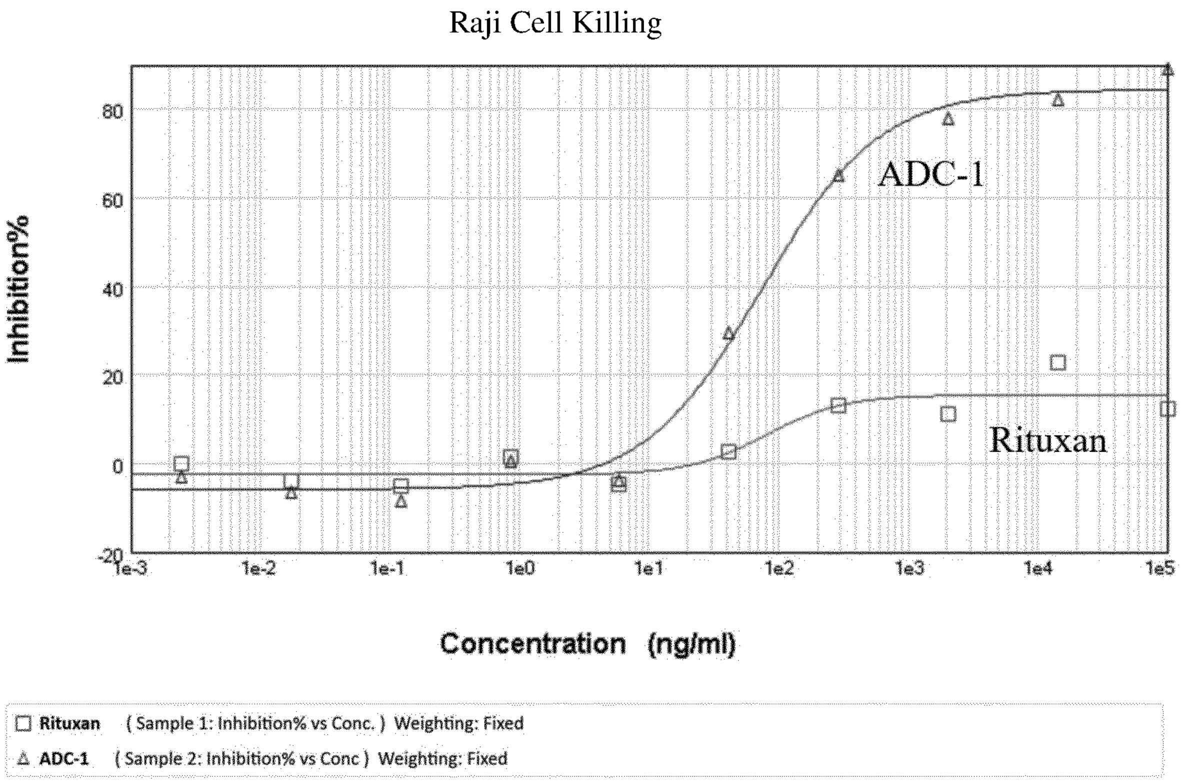


**Figure 4**

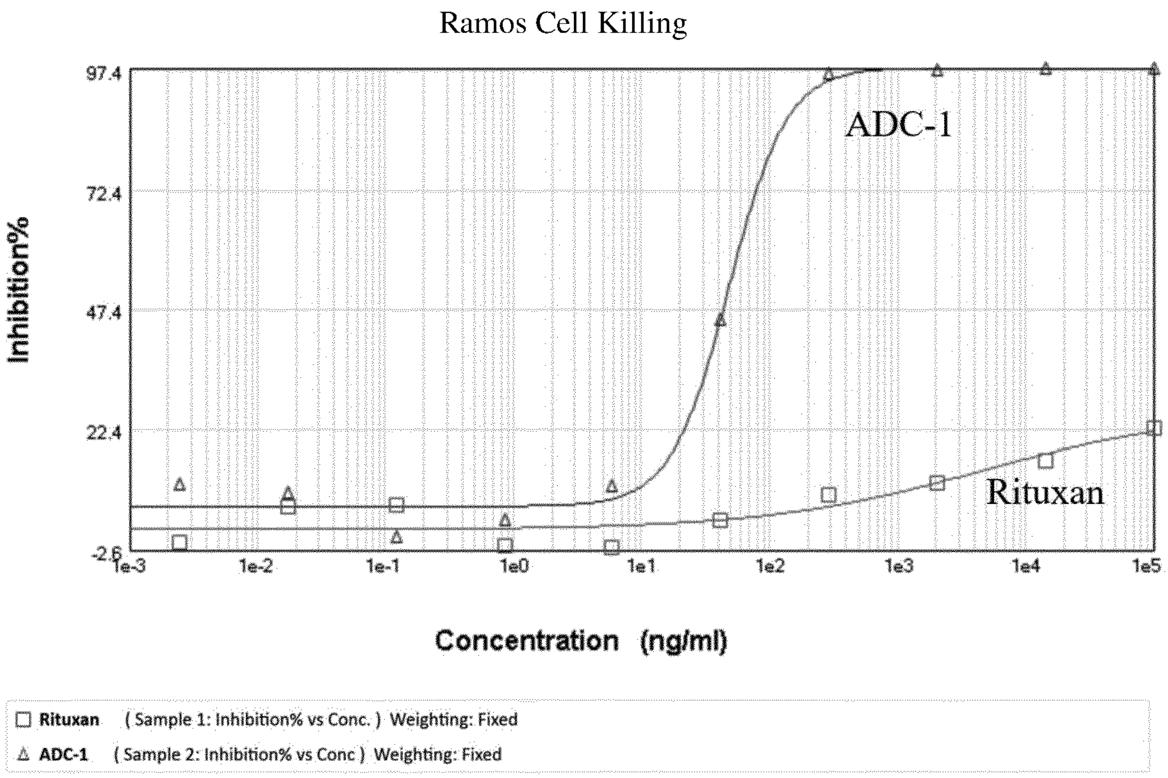


**Figure 5**

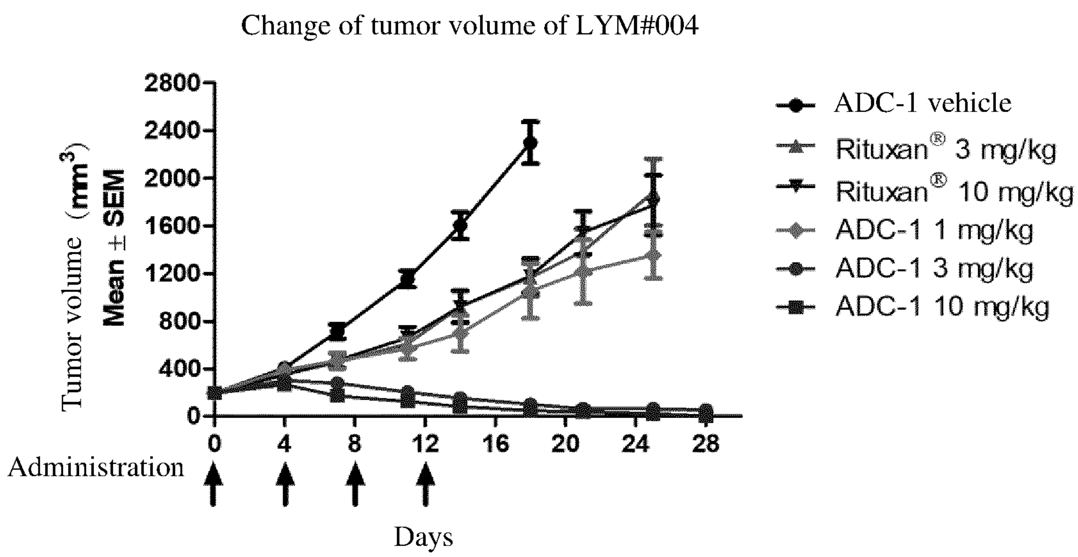


**Figure 6**

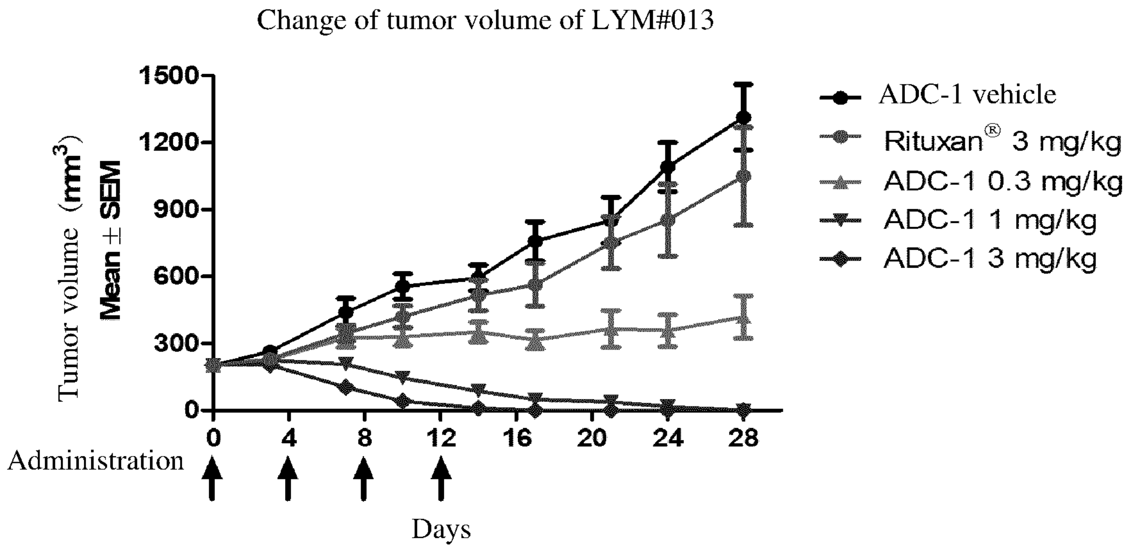


**Figure 7**

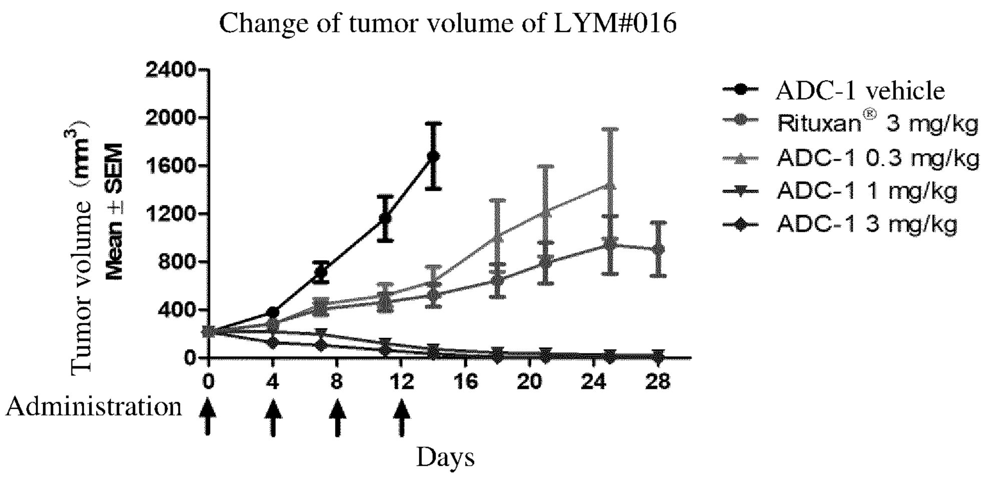


**Figure 8**

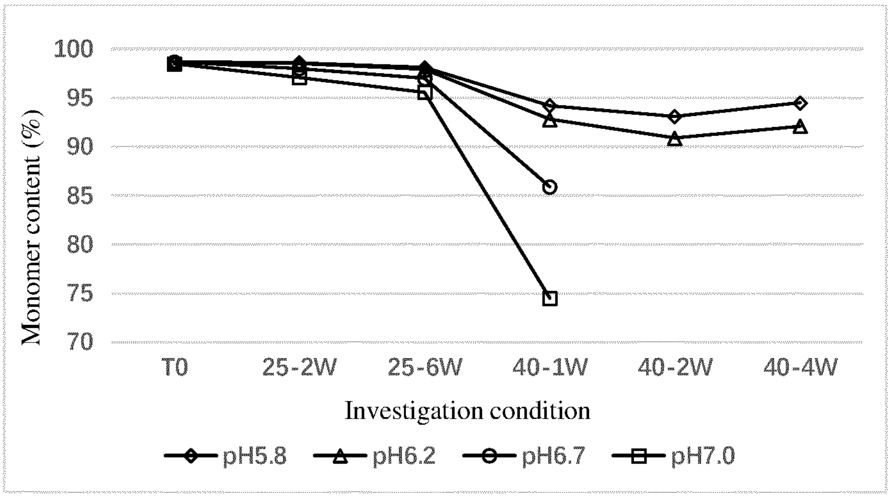
100
95
90
85
80
75
70
Monomer content (%)
T0
25-2W
25-6W
40-1W
40-2W
40-4W
Investigation condition
pH5.8
pH6.2
pH6.7
pH7.0

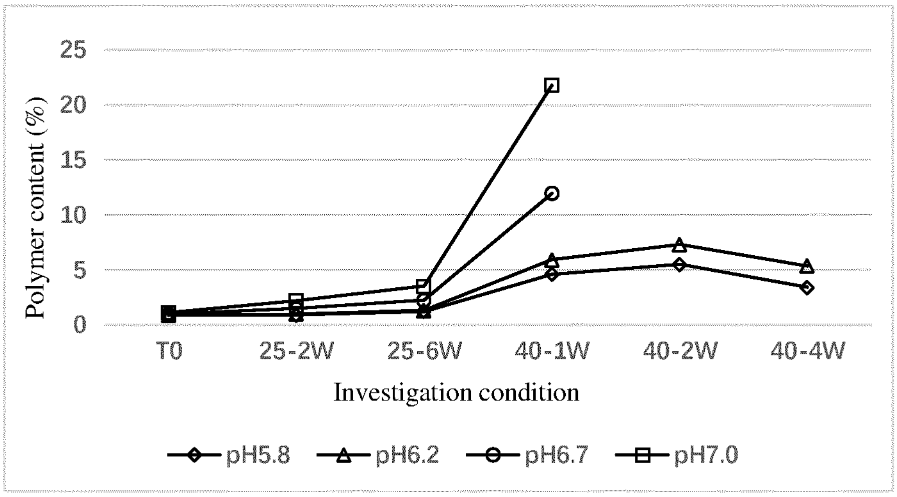
25
20
15
10
5
0
Polymer content (%)
T0
25-2W
25-6W
40-1W
40-2W
40-4W
Investigation condition
pH5.8
pH6.2
pH6.7
pH7.0

**Figure 9**

**INTERNATIONAL SEARCH REPORT**

International application No.
**PCT/CN2020/088564**

**A. CLASSIFICATION OF SUBJECT MATTER**

C07K 19/00(2006.01)i； C07K 1/107(2006.01)i； A61K 38/07(2006.01)i； A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, CNKI, CNTXT, DWPI, SIPOABS, EPTXT, USTXT, WOTXT, JPTXT, NCBI and Keywords: 抗体偶联药物, ADC, CD20抗体, Monomethyl auristatin E, MMAE, 甲基奥利斯达汀E, 细胞毒剂, 6-马来酰亚氨基己酰基-缬氨酸-瓜氨酸-对氨基苄氧羰基, MC-vc-PAB, antibody drug conjugate, ADC

**C. DOCUMENTS CONSIDERED TO BE RELEVANT**

| Category* | Citation of document, with indication, where appropriate, of the relevant passages | Relevant to claim No. |
|---|---|---|
| Y | MEGHDAD, A. A. et al. "A Developed Antibody–Drug Conjugate RituximabvcMMAE shows a Potent Cytotoxic Activity against CD20-positive Cell Line" *Artificial Cells, Nanomedicine, and Biotechnology,* Vol. 46, No. S1, 05 November 2018 (2018-11-05), the abstract, Materials and methods, page S6, left column, paragraph 1, and figure 1 | 1-7, 11-13 |
| Y | CN 103254317 A (ZHEJIANG UNIVERSITY) 21 August 2013 (2013-08-21) claims 1-10, and embodiments | 1-7, 11-13 |
| A | LAW, C. L. et al. "Efficient Elimination of B-lineage Lymphomas by Anti-CD20-Auristatin Conjugates" *Clinical Cancer Research,* Vol. 10, No. 23, 01 December 2004 (2004-12-01), ISSN: 1078-0432, the abstract, and Materials and Methods | 1-7, 11-13 |
| A | PAN, L. Q. et al. "Sortase A-Generated Highly Potent Anti-CD20-MMAE Conjugates for Efficient Elimination of B-Lineage Lymphomas" *Small,* Vol. 13, No. 6, 15 February 2017 (2017-02-15), ISSN: 1078-0432, the abstract, and p. 2, right column, paragraph 1 | 1-7, 11-13 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 January 2021** | **18 February 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/088564**

**C. DOCUMENTS CONSIDERED TO BE RELEVANT**

| Category* | Citation of document, with indication, where appropriate, of the relevant passages | Relevant to claim No. |
|---|---|---|
| A | CN 103145847 A (ZHEJIANG UNIVERSITY) 12 June 2013 (2013-06-12) claims 1-8, and description, paragraphs [0005] and [0012]-[0018] | 1-7, 11-13 |

Form PCT/ISA/210 (second sheet) (January 2015)

**INTERNATIONAL SEARCH REPORT**

International application No.
**PCT/CN2020/088564**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:

[1] PCT Rule 39.1(iv) - Methods for the treatment of the human or animal body by therapy

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/088564**

| Patent document cited in search report | Publication date (day/month/year) | Patent family member(s) | Publication date (day/month/year) |
|---|---|---|---|
| CN 103254317 A | 21 August 2013 | CN 103254317 B | 13 August 2014 |
| CN 103145847 A | 12 June 2013 | CN 103145847 B | 21 May 2014 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5736137 A


### Non-patent literature cited in the description


- **KABAT.** Sequences of Proteins of Immunological Interest. National Institutes of Health, 1987
- **CHOTHIA ; LESK.** *J. Mol. Biol.,* 1987, vol. 196, 901-917
- **CHOTHIA et al.** *Nature,* 1989, vol. 342, 878-883